(19) Europäisches Patentamt
European Patent Office
Office européen des brevets

(11) EP 4 729 058 A1

(12) # EUROPEAN PATENT APPLICATION
published in accordance with Art. 153(4) EPC

(43) Date of publication:
**22.04.2026 Bulletin 2026/17**

(21) Application number: **24823458.5**

(22) Date of filing: **14.06.2024**

(51) International Patent Classification (IPC):
***A61K 31/4015*** *(2006.01)* ***A23L 33/10*** *(2016.01)*
***A61P 3/00*** *(2006.01)* ***A61P 3/04*** *(2006.01)*
***A61P 3/06*** *(2006.01)* ***A61P 21/00*** *(2006.01)*

(52) Cooperative Patent Classification (CPC):
**A23L 33/10; A61K 31/4015; A61P 3/00; A61P 3/04; A61P 3/06; A61P 21/00**

(86) International application number:
**PCT/JP2024/021602**

(87) International publication number:
**WO 2024/257845 (19.12.2024 Gazette 2024/51)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **16.06.2023 JP 2023099429**

(71) Applicant: **Otsuka Pharmaceutical Factory, Inc.**
**Naruto-shi, Tokushima 772-8601 (JP)**

(72) Inventors:
- **YAMAOKA, Ippei**
  **Naruto-shi, Tokushima 772-8601 (JP)**
- **KAGAWA, Tomohiro**
  **Naruto-shi, Tokushima 772-8601 (JP)**
- **MIKI, Yukari**
  **Naruto-shi, Tokushima 772-8601 (JP)**

(74) Representative: **D Young & Co LLP**
**3 Noble Street**
**London EC2V 7BQ (GB)**

(54) **AGENT FOR PROMOTING ENERGY CONSUMPTION**

(57) An object of the present invention is to provide a superior agent that has an energy consumption promoting effect, suppresses an increase in body weight or body fat percentage, and/or can contribute to, for example, prevention or amelioration of obesity, as well as a pharmaceutical composition, a food composition, or the like containing the agent. The above object can be solved by using a compound of the following formula (A).

[Chem. 1]

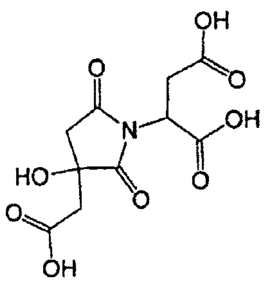

(A)

[Fig. 4]

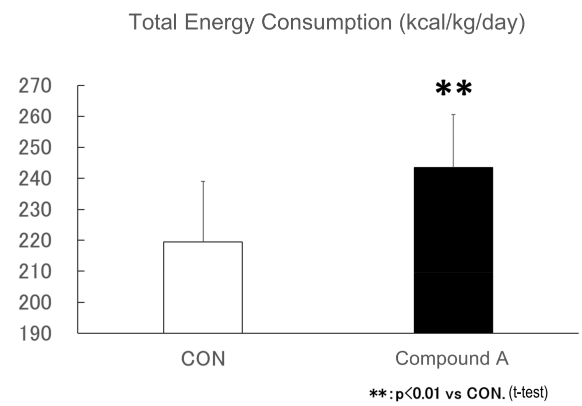


Data are mean ± SD , CON: n = 10, Compound A: n=10.
** p < 0.01; Compound A vs CON.(t-test)

EP 4 729 058 A1

## Description

### Technical Field

The present invention relates to an agent for promoting energy consumption, an agent for promoting lipid burning, an agent for promoting thermogenesis, an agent for suppressing an increase in body weight, an agent for suppressing an increase in or for reducing body fat percentage, an agent for enhancing muscle, an agent for preventing or ameliorating obesity, an agent for suppressing an increase in or for reducing total cholesterol level, and the like.

### Background Art

Obesity is a condition wherein energy intake from sugar, lipid, and the like is excessively accumulated as fat under the skin and around the internal organs, and the body weight is greater than normal level. In recent years, the concept of metabolic syndrome has been attracting attention, which is based on excessive accumulation of visceral fat (obesity) and is a condition that predisposes to arteriosclerosis complicated by glucose intolerance (hyperglycemia), a high neutral fat level (hyperlipidemia), and high blood pressure, or that is to say, a condition involving an increased risk of contracting lifestyle-related diseases.

In recent years, the number of people suffering from obesity and metabolic syndrome has been increasing due to overnutrition and lack of physical exercise. The increased number of people suffering from obesity and metabolic syndrome may lead to increased medical costs, and thus obesity and metabolic syndrome are being a major problem not only in Japan but also on a global scale.

Obesity is induced by energy intake that is greater than energy consumption and, accordingly, a possible method for ameliorating obesity may be reducing energy intake from lipid and the like, or promoting the metabolism of the body in some way to increase energy consumption.

Substances derived from natural products, such as a rice bran extract (patent document 1) and a licorice extract (patent document 2), have been reported to promote energy consumption. Substances derived from natural products may be preferable in terms of safety for long-term consumption, but are problematic by likely having unstable quality and impurity-related issues. Accordingly, compounds that can be chemically synthesized are more readily usable as active ingredients. Such compounds, however, are required to be capable of being inexpensively synthesized in large amounts in consideration of their industrial use. In addition, among synthetic compounds, those that have not been consumed before may raise safety concerns.

Meanwhile, a compound of the following formula (A) is known, and this compound is also known to have an effect of suppressing hepatic disorders such as non-alcoholic steatohepatitis (NASH) (patent document 3). This compound is a substance contained in the neutralized product of a Japanese apricot flesh extract and is thus widely consumed, and techniques for inexpensive, large-scale synthesis and separation/purification are established (patent document 4). Accordingly, it is suitable for industrial use as a component of food, pharmaceutical, and the like.

[Chem. 1]

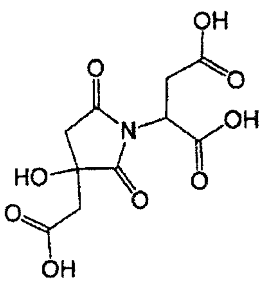

(A)

However, other effects of the compound are not known.

### Prior Art Documents

### Patent Documents

[Patent Document 1]
Japanese unexamined Patent Application Publication No. 2012-020941
[Patent Document 2]
Japanese unexamined Patent Application Publication No. 2018-58832
[Patent Document 3]
WO2017/077707
[Patent Document 4]
WO2018/199040

**Summary of the Invention**

**Object to be Solved by the Invention**

An object of the present invention is to provide a superior agent that has an energy consumption promoting effect, suppresses an increase in body weight or body fat percentage, and/or can contribute to, for example, prevention or amelioration of obesity, as well as a pharmaceutical composition, a food composition, or the like containing the agent.

**Means to Solve the Object**

The present inventors found that the compound of formula (A) (hereinafter also referred to as "compound A") below has an energy consumption promoting effect, suppresses an increase in body weight or body fat percentage, and can contribute to, for example, prevention or amelioration of obesity, and accomplished the present invention.

[Chem. 2]

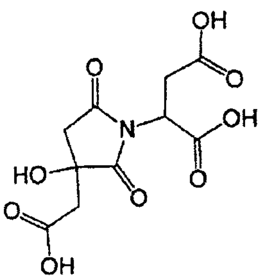

(A)

That is to say, the present invention is identified by the following items:

[1] An agent for promoting energy consumption, comprising a compound of the following formula (A) or a salt thereof as an active ingredient.

[Chem. 3]

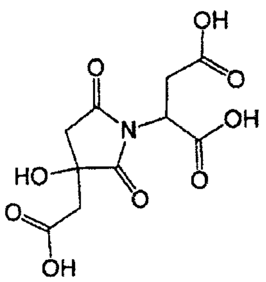

(A)

[2] The agent for promoting energy consumption according to "1", wherein the agent promotes energy consumption by increasing oxygen consumption.
[3] An agent for promoting lipid burning, comprising a compound of the following formula (A) or a salt thereof as an

active ingredient.

[Chem. 4]

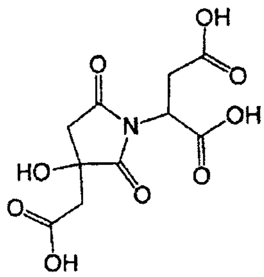

(A)

[4] The agent for promoting lipid burning according to "3", wherein the agent is for promoting fat burning.
[5] An agent for promoting thermogenesis, comprising a compound of the following formula (A) or a salt thereof as an active ingredient.

[Chem. 5]

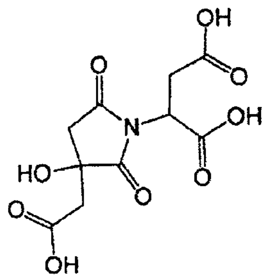

(A)

[6] The agent for promoting thermogenesis according to "5", wherein the agent is for increasing body temperature.
[7] An agent for suppressing an increase in body weight, comprising a compound of the following formula (A) or a salt thereof as an active ingredient.

[Chem. 6]

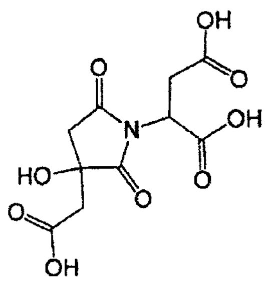

(A)

[8] A n agent for suppressing an increase in or for reducing body fat percentage, comprising a compound of the following formula (A) or a salt thereof as an active ingredient.

[Chem. 7]

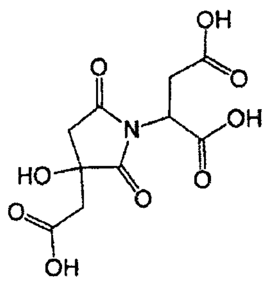

(A)

[9] The agent for suppressing an increase in or for reducing body fat percentage according to "8", wherein the agent is for suppressing an increase in body fat mass.
[10] The agent for suppressing an increase in or for reducing body fat percentage according to "8", wherein the agent is for suppressing body fat accumulation.
[11] An agent for enhancing muscle, comprising a compound of the following formula (A) or a salt thereof as an active ingredient.

[Chem. 8]

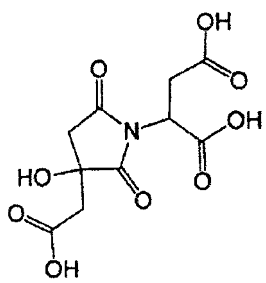

(A)

[12] The agent for enhancing muscle according to "11", wherein the agent is for promoting an increase in muscle mass.
[13] An agent for preventing or ameliorating obesity, comprising a compound of the following formula (A) or a salt thereof as an active ingredient.

[Chem. 9]

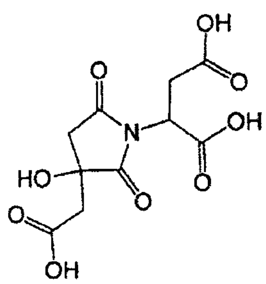

(A)

[14] An agent for suppressing an increase in or for reducing total cholesterol level, comprising a compound of the following formula (A) or a salt thereof as an active ingredient.

[Chem. 10]

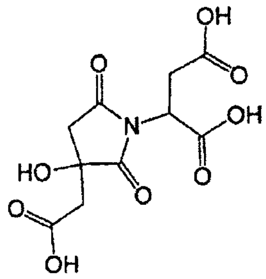

(A)

[15] A pharmaceutical composition or a food composition, comprising the agent according to any one of "1" to "14".

Other embodiments of the present invention may be as follows:

1) Use of compound A or a salt thereof for the manufacture of an agent for promoting energy consumption, an agent for increasing oxygen consumption , an agent for promoting lipid burning, an agent for promoting fat burning, an agent for promoting thermogenesis, an agent for increasing body temperature, an agent for suppressing an increase in body weight, an agent for suppressing an increase in or for reducing body fat percentage, an agent for suppressing an increase in body fat mass, an agent for suppressing body fat accumulation, an agent for enhancing muscle, an agent for promoting an increase in muscle mass, an agent for preventing or ameliorating obesity, an agent for suppressing an increase in or for reducing total cholesterol level, or the like.
2-1) Compound A or a salt thereof for use in promoting energy consumption, increasing oxygen consumption, promoting lipid burning, promoting fat burning, promoting thermogenesis, increasing body temperature, suppressing an increase in body weight, suppressing an increase in or reducing body fat percentage, suppressing an increase in body fat mass, suppressing body fat accumulation, enhancing muscle, promoting an increase in muscle mass, preventing or ameliorating obesity, or suppressing an increase in or reducing total cholesterol level.
2-2) Compound A or a salt thereof for use in preventing or treating hypothermia or sensitivity to cold.
2-3) Compound A or a salt thereof for use in preventing or treating obesity or metabolic syndrome.
2-4) Compound A or a salt thereof for use in preventing or treating a pathological condition or a disease caused by obesity or metabolic syndrome.
2-5) Compound A or a salt thereof for use in preventing or treating dyslipidemia.
2-6) Compound A or a salt thereof for use in preventing or treating a pathological condition or a disease caused by an increased total cholesterol level.
3-1) a method for promoting energy consumption, a method for increasing oxygen consumption, a method for promoting lipid burning, a method for promoting fat burning, a method for promoting thermogenesis, a method for increasing body temperature, a method for suppressing an increase in body weight, a method for suppressing an increase in or for reducing body fat percentage, a method for suppressing an increase in body fat mass, a method for suppressing body fat accumulation, a method for enhancing muscle, a method for promoting an increase in muscle mass, a method for preventing or ameliorating obesity, a method for suppressing an increase in or for reducing total cholesterol level, comprising administering an effective amount of compound A or a salt thereof to a subject in need thereof.
3-2) A method for preventing or a method for treating hypothermia or sensitivity to cold, comprising administering an effective amount of compound A or a salt thereof to a subject in need of preventing or treating hypothermia or sensitivity to cold.
3-3) A method for preventing or a method for treating obesity or metabolic syndrome, comprising administering an effective amount of compound A or a salt thereof to a subject in need of preventing or treating obesity or metabolic syndrome.
3-4) A method for preventing or a method for treating a pathological condition or a disease caused by obesity or metabolic syndrome, comprising administering an effective amount of compound A or a salt thereof to a subject in need of preventing or treating a pathological condition or a disease caused by obesity or metabolic syndrome.
3-5) A method for preventing or a method for treating dyslipidemia, comprising administering an effective amount of compound A or a salt thereof to a subject in need of preventing or treating dyslipidemia.
3-6) A method for preventing or a method for treating a pathological condition or a disease caused by an increased total cholesterol level, comprising administering an effective amount of compound A or a salt thereof to a subject in

need of preventing or treating a pathological condition or a disease caused by an increased total cholesterol level.

## Effect of the Invention

According to the present invention, it is possible to provide an agent, a food composition, a pharmaceutical composition, and the like useful for, for example, promoting energy consumption, promoting lipid burning, promoting thermogenesis, suppressing an increase in body weight, suppressing an increase in or reducing body fat percentage, enhancing muscle, preventing or ameliorating obesity, and/or suppressing an increase in or reducing total cholesterol level.

## Brief Description of Drawings

[Figure 1] A graph illustrating the daily calorie intake during the test period. In the figure, "CON" denotes the "CON group" set forth in the Examples, and "compound A" denotes the "compound A administered group" set forth in the Examples. The same applies to the following figures.
[Figure 2] A graph illustrating the total oxygen consumption over 24 hours after administration in the morning on day 82 from the start of the test.
[Figure 3] A graph illustrating the total carbon dioxide production over 24 hours after administration in the morning on day 82 from the start of the test.
[Figure 4] A graph illustrating the total energy consumption over 24 hours after administration in the morning on day 82 from the start of the test.
[Figure 5] A graph illustrating the total lipid burning over 24 hours after administration in the morning on day 82 from the start of the test.
[Figure 6] A graph illustrating the rectal temperature before necropsy.
[Figure 7] A graph illustrating body weight changes during the test food administration period.
[Figure 8] Graphs illustrating body fat percentage and lean body mass percentage after 6 weeks of test food intake, after 9 weeks of test food intake, and after 12 weeks of test food intake.
[Figure 9] Graphs illustrating the white fat weight (epididymal fat, mesenteric fat, perirenal fat, and interscapular fat) per body weight at the time of necropsy.
[Figure 10] Graphs illustrating the respective weights of the plantaris muscle, anterior tibial muscle, and extensor digitorum longus muscle, as well as the total weight of these three types of muscle per body weight at the time of necropsy.
[Figure 11] A graph illustrating the total cholesterol level in plasma at the time of necropsy.

## Mode of Carrying Out the Invention

An agent for promoting energy consumption, an agent for promoting lipid burning, an agent for promoting thermogenesis, an agent for suppressing an increase in body weight, an agent for suppressing an increase in or for reducing body fat percentage, an agent for enhancing muscle, an agent for preventing or ameliorating obesity, and an agent for suppressing an increase in or for reducing total cholesterol level of the present invention (hereinafter these may be collectively referred to as the "agent of the present invention") comprise compound A or a salt thereof as an active ingredient.

Here, "comprising as an active ingredient" means comprising an effective amount. The effective amount means an amount sufficient to exert the desired effect in vivo. In addition, in the present invention, "as an active ingredient" means that an ingredient other than compound A or a salt thereof may be comprised as long as the effects of the present invention are not impaired. The effective amount may vary according to the condition of a subject, severity of a pathological condition or a disease, animal species, age (age in month), sex, or body weight of the subject, or the like.

(Compound A)

Compound A used in the present invention is a compound of the following formula (A).

[Chem. 11]

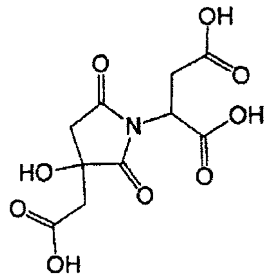

(A)

Compound A has two asymmetric carbon atoms and thus has four stereoisomers (that is, compound A having a steric structure of SS isomer, SR isomer, RS isomer or RR isomer in the RS notation system), while in the present invention, it is not particularly limited which stereoisomer to use. That is to say, in the present invention, any stereoisomer may be used alone, or among the stereoisomers, a mixture comprising a combination of any two, three, or four isomers at any proportions may be used. In this regard, since compounds A having steric structures of SS isomer and RR isomer in the RS notation system are crystalline (WO2018/199040) and thus easier to handle, it is preferred to use as the compound A in the present invention, compound A having the steric structure of SS isomer or RR isomer in the RS notation system or to use a mixture of compound A having the steric structure of SS isomer and compound A having the steric structure of RR isomer in the RS notation system.

Compound A is known to be contained in Japanese apricot, a Japanese apricot extract, a neutralized product of a Japanese apricot extract, and the like. Thus, compound A may be separated/purified therefrom by any method and used, or chemically synthesized and used, or a commercially available product thereof may be purchased and used. However, the amount of compound A contained in Japanese apricot, a Japanese apricot extract, a neutralized product of a Japanese apricot extract, and the like is limited, thus it may be inefficient to use as an active ingredient of the agent of the present invention. Furthermore, substances derived from natural products may have unstable quality or impurity issues. Therefore, chemically synthesized compound A or a salt thereof is preferably used. That is to say, the agent of the present invention may be in an embodiment that is free of or substantially free of Japanese apricot, a Japanese apricot extract, a neutralized product of a Japanese apricot extract, and the like. Also, the agent of the present invention may be in an embodiment that is free of or substantially free of another natural product (e.g., a *Rosaceae* plant), natural product extract (e.g., a *Rosaceae* plant extract), neutralized product of a natural product (e.g., a neutralized product of a *Rosaceae* plant extract), and the like. Here, being "substantially free" means that the agent does not comprise an effective amount and/or may comprise an amount lower than an effective amount.

The method for chemically synthesizing, separating, or purifying compound A or a salt thereof is not particularly limited, and, for example, compound A can be obtained by reacting citric acid with L-aspartic acid. When chemically synthesizing and separating/purifying compound A or a salt thereof, a method described in, for example, WO2017/07707 or WO2018/19940 is preferable because purified (highly purified) compound A or a salt thereof can be inexpensively obtained in large amounts.

(Salt of compound A)

The salt of compound A used in the present invention may be a metal salt or an amino acid salt of compound A.

The metal salt may be a metal salt selected from the group consisting of a sodium salt, a potassium salt, a magnesium salt, and a calcium salt, and a calcium salt is preferable.

The amino acid salt may be an amino acid salt selected from the group consisting of an arginine salt, a citrulline salt, an ornithine salt, and a histidine salt. The amino acid salt is preferably an L-amino acid salt.

(Agent for promoting energy consumption and agent for increasing oxygen consumption)

Compound A or a salt thereof has an energy consumption promoting effect and thus can be suitably used as an active ingredient of an agent for promoting energy consumption.

"Energy consumption" in the present invention means that nutrients (energy sources) such as lipid, protein, and carbohydrate are metabolized in various tissues of the body and converted to chemical energy or thermal energy. "Metabolism" encompasses "burning" or "oxidation".

"Promoting energy consumption" means increasing energy consumption, such as increasing the total energy

consumption over a certain period of time after administration of compound A. For example, the total energy consumption over 24 hours can be calculated from the mean oxygen consumption and the mean carbon dioxide production over about 24 hours (e.g., 24 hours ± 1 hour) using the following formula:

Total energy consumption (kcal/kg/day) = [{3.816 × Mean $VO_2$ over about 24 hours (mL/min) + 1.231 × Mean $VCO_2$ over about 24 hours (mL/min) } / Body weight (kg) × 60 (min/h) × 24 (h))] / 1000 (cal/kcal)

($VO_2$: oxygen consumption, $VCO_2$: carbon dioxide production)

When compound A or a salt thereof is administered, the total energy consumption over a certain period of time (e.g., 24 hours) can be increased compared with the case where it is not administered. The increase may be an increase of preferably 3% or more, more preferably 5% or more, even more preferably 7% or more, and particularly preferably 10% or more. Also, the increase may be an increase of preferably 20% or less, more preferably 18% or less, and particularly preferably 15% or less. Accordingly, the increase may be an increase of 3 to 20%, 5 to 20%, 7 to 20%, 10 to 20%, 3 to 18%, 5 to 18%, 7 to 18%, 10 to 18%, 3 to 15%, 5 to 15%, 7 to 15%, and 10 to 15%, and the like.

Oxygen is required during the course of energy consumption process, and thus the agent for promoting energy consumption of the present invention can also be used as an agent for promoting energy consumption wherein the promotion is by increasing oxygen consumption (an agent for promoting energy consumption wherein the agent promotes energy consumption by increasing oxygen consumption). That is to say, compound A or a salt thereof can also be suitably used as an active ingredient of an agent for increasing oxygen consumption or an agent for promoting oxygen consumption. The method for calculating oxygen consumption is not particularly limited, and can be measured with, for example, a commercially available apparatus capable of measuring exhaled gas. Also, oxygen consumption may be evaluated as a total oxygen consumption over a certain period of time. For example, the total oxygen consumption over 24 hours can be calculated from the mean oxygen consumption over about 24 hours (e.g., 24 hours ± 1 hour) using the formula shown below:

Total oxygen consumption (mL/kg/day) = Mean $VO_2$ over about 24 hours (mL/min) / Body weight (kg) × 60 (min/h) × 24 (h)

($VO_2$: Oxygen consumption)

"Increasing oxygen consumption " or "promoting oxygen consumption" means increasing oxygen consumption. When compound A or a salt thereof is administered, oxygen consumption over a certain period of time (e.g., 24 hours) can be increased compared with the case where it is not administered. The increase may be an increase of preferably 3% or more, more preferably 5% or more, even more preferably 7% or more, and particularly preferably 10% or more. Also, the increase may be an increase of preferably 20% or less, more preferably 18% or less, and particularly preferably 15% or less. Accordingly, the increase may be an increase of 3 to 20%, 5 to 20%, 7 to 20%, 10 to 20%, 3 to 18%, 5 to 18%, 7 to 18%, 10 to 18%, 3 to 15%, 5 to 15%, 7 to 15%, and 10 to 15%, and the like.

(Agent for promoting lipid burning)

Compound A or a salt thereof has a lipid burning promoting effect and thus can be suitably used as an active ingredient of an agent for promoting lipid burning.

"Lipid burning" in the present invention means that lipid derived from food or derived from fat accumulated in the body, especially fatty acid, is metabolized in various tissues of the body and converted to chemical energy or thermal energy.

"Promoting lipid burning" means increasing lipid burning and, for example, means increasing the total lipid burning over a certain period of time after compound A is administered. For example, the total lipid burning over 24 hours can be calculated from the mean oxygen consumption and the mean carbon dioxide production over about 24 hours (e.g., 24 hours ± 1 hour) using the formula shown below:

Total lipid burning (mg/kg/day) = 1.67 × [Mean $VO_2$ over about 24 hours (mL/min) - Mean $VCO_2$ over about 24 hours (mL/min)] / Body weight (kg) × 60 (min/h) × 24 (h)

($VO_2$: oxygen consumption, $VCO_2$: carbon dioxide production)

When compound A or a salt thereof is administered, the total fat burning over a certain period of time (e.g., 24 hours) can be increased compared with the case where it is not administered. The increase may be an increase of preferably 1% or more, more preferably 2% or more, and even more preferably 3% or more. Also, the increase may be an increase of preferably 10% or less, more preferably 8% or less, and even more preferably 5% or less. Accordingly, the increase may be an increase of 1 to 10%, 2 to 10%, 3 to 10%, 1 to 8%, 2 to 8%, 3 to 8%, 1 to 5%, 2 to 5%, and 3 to 5%, and the

like.

The agent for promoting lipid burning of the present invention can also be suitably used to promote fat burning (especially white fat) accumulated in the body. That is to say, compound A or a salt thereof can also be suitably used as an active ingredient of the agent for promoting fat burning.

(Agent for promoting thermogenesis)

As described above, compound A or a salt thereof has an energy consumption promoting effect, and when energy consumption is promoted in the body, thermogenesis is also promoted. Accordingly, compound A or a salt thereof has a thermogenesis promoting effect, and can be suitably used as an active ingredient of an agent for promoting thermogenesis.

"Thermogenesis" in the present invention means that the body produces heat, i.e., energy, due to metabolism being more activated than usual.

The method for evaluating thermogenesis is not particularly limited, and thermogenesis can be evaluated by, for example, measuring the body temperature. Note that the body temperature may be the temperature of any part of the body, such as the temperature of the rectum, axilla, mouth, ear.

The body temperature can increase when thermogenesis is promoted in the body, and thus the agent for promoting thermogenesis of the present invention can also be used to increase body temperature. That is to say, compound A or a salt thereof can also be suitably used as an active ingredient of an agent for increasing body temperature. The agent for promoting thermogenesis or the agent for increasing body temperature of the present invention may be used to prevent or ameliorate hypothermia or physical chill (e.g., sensitivity to cold).

For example, when compound A or a salt thereof is administered, the rectal temperature after a lapse of a certain period of time (e.g., administration of twice per day for 12 weeks) can be increased compared with the case where it is not administered. The increase may be an increase of preferably 1% or more, more preferably 2% or more, and even more preferably 3% or more. Also, the increase may be an increase of preferably 10% or less, more preferably 9% or less, and even more preferably 7% or less. Accordingly, the increase may be an increase of 1 to 10%, 2 to 10%, 3 to 10%, 1 to 9%, 2 to 9%, 3 to 9%, 1 to 7%, 2 to 7%, and 3 to 7%, and the like.

The increase may be an increase of preferably 0.1°C or more, more preferably 0.2°C or more, and even more preferably 0.3°C or more. Also, the increase may be an increase of preferably 3°C or less, more preferably 2.8°C or less, and even more preferably 2.5°C or less. Accordingly, the increase may be an increase of 0.1 to 3°C, 0.2 to 3°C, 0.3 to 3°C, 0.1 to 2.8°C, 0.2 to 2.8°C, 0.3 to 2.8°C, 0.1 to 2.5°C, 0.2 to 2.5°C, and 0.3 to 2.5°C, and the like.

(Agent for suppressing an increase in body weight)

Compound A or a salt thereof has a body weight increase suppressing effect and thus can be suitably used as an active ingredient of an agent for suppressing an increase in body weight. The agent for suppressing an increase in body weight of the present invention may also be used as an agent for reducing body weight.

The method for calculating the body weight is not particularly limited, and can be calculated with, for example, a commercially available scale or body composition analyzer.

For example, compared with the increase in body weight over a certain period of time (e.g., 12 weeks) when compound A or a salt thereof is not administered being 100%, the increase in body weight when compound A or a salt thereof is administered may be 10 to 90%, 20 to 90%, 30 to 90%, 10 to 80%, 20 to 80%, 30 to 80%, 10 to 70%, 20 to 70%, and 30 to 70%, and the like.

(An agent for suppressing an increase in or for reducing body fat percentage)

Compound A or a salt thereof has a body fat percentage or body fat mass (e.g., body fat weight, especially white fat weight, per body weight) increase suppressing effect, and thus can be suitably used as an active ingredient of an agent for suppressing an increase in or for reducing body fat percentage, or an agent for suppressing an increase in or for reducing body fat mass. As the body fat percentage or the body fat mass is decreased, the lean body mass percentage or the lean body mass is increased, and vice versa. Thus, the agent for suppressing an increase in or for reducing body fat percentage, or the agent for suppressing an increase in or for reducing body fat mass, of the present invention can also be suitably used as an agent for suppressing a decrease in or for increasing lean body mass percentage or an agent for suppressing a decrease in or for increasing lean body mass. Also, the agent for suppressing an increase in or for reducing body fat percentage of the present invention suppresses an increase in body fat mass as described above, and thus can be suitably used to suppress body fat accumulation as well. That is to say, compound A or a salt thereof can also be suitably used as an active ingredient of an agent for suppressing body fat accumulation.

"Body fat" refers to fat stored in the body, most of which is neutral fat, and is stored primarily in fat cells. Fat cells

are roughly divided into two types, i.e., so-called white fat cells such as subcutaneous fat and visceral fat, and brown fat cells. White fat cells have unilocular lipid droplets, store excess energy as neutral fat in the lipid droplets, and function, as necessary, to decompose it into glycerol and free fatty acid and resupplying it to the whole body as an energy source. Accordingly, in an obese state wherein energy supply chronically exceeds energy consumption, lipid droplets accumulated in white fat cells become enlarged. Adult fat tissue is mostly white fat tissue. The white fat (tissue) composed of white fat cells is not particularly limited, and examples include epididymal fat, mesenteric fat, perirenal fat, and interscapular fat.

In the present invention, the method for calculating body fat mass (e.g., white fat weight) is not particularly limited, and the body fat mass can be calculated with a commercially available body composition analyzer, computed tomography (CT scan), magnetic resonance imaging (MRI), or the like. Also, the method for calculating body fat percentage is not particularly limited, and the body fat percentage can be calculated by, for example, the same method as the body fat mass or can be calculated as the proportion of body fat mass in the body weight. When an experimental animal is used, the body fat mass may be calculated by removing each fat (e.g., white fat) from the body and directly measuring the weight thereof.

(Agent for enhancing muscle and agent for promoting an increase in muscle mass)

Compound A or a salt thereof can be suitably used as an active ingredient of an agent for enhancing muscle. "Enhancing muscle" in the present invention means enhancing muscle and/or increasing muscle mass. Compound A or a salt thereof has a muscle mass (e.g., the weight of muscle, especially the weight of skeletal muscle, per body weight) increase promoting effect, and thus can also be used to promote increase muscle mass. That is to say, compound A or a salt thereof can also be suitably used as an active ingredient of an agent for promoting an increase in muscle mass.

The muscle is not particularly limited, and may be, for example, the skeletal muscle, especially the plantaris muscle, anterior tibialis muscle, and extensor digitorum longus muscle, which are the left and right lower limb skeletal muscles.

The method for calculating muscle mass (e.g., the weight of skeletal muscle) is not particularly limited, and the muscle mass may be calculated with, for example, a commercially available body composition analyzer, computed tomography (CT scan), magnetic resonance imaging (MRI). When an experimental animal is used, the muscle mass may be calculated by removing each muscle (e.g., skeletal muscle) from the body and directly measuring the weight thereof. Also, the muscle mass may be evaluated, for example, for each muscle exemplified above, or by summing the weights of those muscles. The proportion of muscle in the body weight can also be referred to as muscle percentage or muscle mass percentage. That is to say, compound A or a salt thereof can also be used as an active ingredient of an agent for promoting an increase in muscle percentage or muscle mass percentage. Since muscle strength increases as the muscle mass or the muscle (mass) percentage increases, compound A or a salt thereof can also be suitably used as an active ingredient of an agent for increasing muscle strength or an agent for enhancing muscle strength.

(Agent for preventing or ameliorating obesity)

Compound A or a salt thereof has an energy consumption promoting effect, a fat burning promoting effect, a body fat percentage increase suppressing effect, a body weight increase suppressing effect, and the like as described above, and thus can be suitably used as an active ingredient of an agent for preventing or ameliorating obesity.

"Obesity" refers to a condition in which not only the body weight is large, but also the body fat is excessively accumulated. Body mass index (BMI) = [Body weight (kg)] / [Height (m)$^2$], which is an international standard index, is used to determine the degree of obesity. A BMI of 22.0 is considered normal for both men and women, and obesity is defined as "a body mass index (BMI) of 25 or greater, with excessive accumulation of fat in fat tissue".

Compound A or a salt thereof can also be suitably used as an active ingredient of an agent for preventing or ameliorating metabolic syndrome. Metabolic syndrome is a combination of obesity, high blood pressure, dyslipidemia, hyperglycemia (diabetes), and the like, and is a condition in which a cardiovascular disease is likely developed. Moreover, compound A or a salt thereof can also be suitably used as an agent for preventing or treating a pathological condition or a disease caused by obesity or metabolic syndrome. Examples of the disease caused by obesity or metabolic syndrome include, but are not particularly limited to, arteriosclerosis, a heart disease (such as angina pectoris or myocardial infarction), stroke, and cerebral infarction.

(Agent for suppressing an increase in or for reducing total cholesterol level)

Compound A or a salt thereof has a total cholesterol level increase suppressing effect, and thus can be suitably used as an active ingredient of an agent for suppressing an increase in or for reducing total cholesterol level.

"Total cholesterol level" refers to the total amount obtained by measuring the entirety of cholesterol contained in blood. A high total cholesterol level may be considered as a result of dyslipidemia, which can cause various diseases such as arteriosclerosis, myocardial infarction, and cerebral infarction.

The method for calculating the total cholesterol level is not particularly limited, and the total cholesterol level can be calculated by, for example, collecting blood and measuring the total cholesterol level in blood.

Compound A or a salt thereof can also be suitably used as an agent for preventing or treating a pathological condition or a disease caused by an increased total cholesterol level. Examples of the disease caused by an increased total cholesterol level include, but are not particularly limited to, arteriosclerosis, a heart disease (such as angina pectoris or myocardial infarction), stroke, and cerebral infarction.

(Composition)

Compound A or a salt thereof can be used as-is, and can also be used as a composition by being suitably blended with another ingredient as long as the effects of the present invention are not impaired. That is to say, a composition comprising the agent for promoting energy consumption, the agent for promoting lipid burning, the agent for promoting thermogenesis, the agent for suppressing an increase in body weight, the agent for suppressing an increase in or for reducing body fat percentage, the agent for promoting an increase in muscle mass, the agent for enhancing muscle, the agent for preventing or ameliorating obesity, the agent for suppressing an increase in or for reducing total cholesterol level, or the like of the present invention can be provided. That is to say, the composition of the present invention can be suitably used as a composition for, for example, promoting energy consumption, promoting lipid burning, promoting thermogenesis, suppressing an increase in body weight, suppressing an increase in or reducing body fat percentage, promoting an increase in muscle mass, enhancing muscle, preventing or ameliorating obesity, suppressing an increase in or reducing total cholesterol level. The composition may be preferably a food composition or a pharmaceutical composition. In particular, a food composition is preferable from the viewpoint of daily intake.

(Food composition)

In the present invention, the food encompasses a beverage. The form of the food composition in the present invention is not particularly limited as long as it can be taken by a subject such as a mammal and is suitable for consumption, and examples include solid, liquid, semiliquid, granular, particulate, powdery, capsular, cream, and paste forms.

The food composition of the present invention may comprise a further ingredient according to the form of each food. Here, the further ingredient is not particularly limited as long as the effects of the present invention are not impaired, and examples include various types of protein, sugar, fat, trace element, analeptic, vitamin, and organic acid salt of citric acid, acetic acid or the like. The food in the present invention may suitably comprise or not comprise an additive that is acceptable according to its type and that is commonly used in food, e.g., a sweetener such as aspartame or stevia, an acidulant such as citric acid, malic acid, or tartaric acid, an excipient such as dextrin or starch, a coloring agent, a flavoring agent, a bittering agent, a buffer, a thickening stabilizer, a gelling agent, a stabilizer, a gum base, a binder, a diluent, an emulsifier, a dispersant, a suspending agent, an antioxidant, a preservative, an antiseptic agent, an antifungal agent, a color developer, a bleach, a brightening agent, an enzyme, a seasoning, or a spice extract.

"Food" in the present invention is used in the sense of including health food, functional food, specified health food, a nutritional supplementary food, and food for the sick. In particular, food is suitably provided as food suitable for consumers expecting obesity preventing or ameliorating function or the like, i.e., specified health food. "Specified health food" as used herein refers to food that may be subject to some legal restriction from health perspective when the food is, for example, manufactured or sold for the purpose of preventing or ameliorating obesity.

The above health food and the like may be in the form of ordinary food, and is preferably in the form of a drink or a supplement (such as a pill, a granule, a fine particle, a tablet, a chewable tablet, or a capsule (such as a soft capsule or a hard capsule)).

In the present invention, examples of the food of interest to which compound A or a salt thereof is added include, but are not limited to, a beverage such as a soft drink, a carbonated drink, a nutritional drink, a fruit drink, or a milk-based drink (including a concentrated stock or a powder preparation of such a drink); carbohydrate-containing food such as rice, noodle, bread, or pasta; a Western confection such as a cookie or cake, a Japanese confection such as manju or yokan, and a variety of confections such as cold sweets and refrigerated sweets, e.g., a candy, chewing gum, yogurt, pudding, or jelly; fishery and livestock processed food such as boiled fish paste, tube-shaped boiled fish paste, hamburger patty, ham, or sausage; a dairy product such as processed milk, fermented milk, yogurt, butter, or cheese; oil/fat and oil/fat processed food such as margarine, mayonnaise, shortening, whipped cream, or dressing; and seasoning such as sauce or dip.

(Pharmaceutical composition)

In the present invention, the pharmaceutical encompasses a quasi-pharmaceutical. The pharmaceutical composition of the present invention may or may not comprise a suitable additive that is pharmaceutically acceptable

and is suitably selected according to the dosage form (such as a carrier, an excipient, a diluent, a binder, a lubricant, a disintegrant or disintegration aid, a solubilizer, a stabilizer, a preservative, an antiseptic agent, a filler, a thickener, an emulsifier, a dispersant, a suspending agent, or a buffer), and can be provided by being prepared into a variety of formulations that can be topically or locally administered in an oral or parenteral manner by a variety of known methods.

The drug composition of the present invention can be orally or parenterally administered. For oral administration, the drug of the present invention may be formulated into a tablet (including a sugar-coated tablet), a capsule, a granule, a powder, a pill, an oral solution, a suspension, an emulsion, a syrup, or the like, or may be formulated into a dry product that is redissolved immediately before use. For parenteral administration, the drug of the present invention is formulated into an injection (such as a subcutaneous injection, an intravenous injection, an intramuscular injection, or an intraperitoneal injection), a drop, a suppository (such as a rectal suppository or a vaginal suppository), or the like, and, in the case of an injectable formulation, is provided in a unit-dose ampoule or a multi-dose container.

Here, the formulation can be produced, for example, as follows. An oral formulation can be produced by adding an excipient (such as lactose, sucrose, starch, or mannitol), a disintegrant (such as calcium carbonate or carboxymethylcellulose calcium), a binder (such as pregelatinized starch, gum arabic, carboxymethylcellulose, polyvinylpyrrolidone, or hydroxypropylcellulose), or a lubricant (such as talc, magnesium stearate, or polyethylene glycol 6000) to the active ingredient, compression-molding the mixture and then, as necessary, applying a coating by a method known per-se to mask the taste and impart enteric properties and sustainability. Examples of usable coating agents include ethyl cellulose, hydroxymethylcellulose, polyoxyethylene glycol, cellulose acetate phthalate, hydroxypropyl methylcellulose phthalate, and Eudragit (a methacrylic acid/acrylic acid copolymer, manufactured by Rohm GmbH, Germany)

(Content in composition)

In a composition such as the above food composition or pharmaceutical composition, the content of compound A or a salt thereof may be, for example, 0.01% by mass or more, 0.1% by mass or more, 1.0% by mass or more, 95% by mass or less, 80% by mass or less, or 60% by mass or less of the total mass of the composition. Also, the content may be 0.01 to 95% by mass, 0.1 to 80% by mass, or 1.0% by mass to 60% by mass.

(Dosage)

In the present invention, the dosage of compound A or a salt thereof as an active ingredient is not particularly limited as long as the effects of the present invention are not impaired, and is in a range of, for example, 0.6 mg to 36000 mg/day, preferably 30 mg to 720 mg/day (adult), and most preferably in a range of 60 to 360 mg/day (adult). Note that the dosage may be suitably determined according to, for example, the age (age in month), body weight, sex, symptom, sensitivity to compound A or a salt thereof of the subject of administration.

(Administration method)

The method for administering the agent or composition of the present invention comprising compound A or a salt thereof is not particularly limited as long as the effects of the present invention are not impaired, and examples of the period thereof include a period of, for example, 1 day or more, 3 days or more, 5 days or more, 1 week or more, 2 weeks or more, 3 weeks or more, 4 weeks or more, 5 weeks or more, 6 weeks or more, and 7 weeks or more, preferably 8 weeks or more and 9 weeks or more, more preferably 10 weeks or more and 11 weeks or more, and even more preferably 12 weeks or more. Continuous use over a long period of time can provide greater effects. The frequency of administration per day is not particularly limited, and may be, for example, once a day or a divided dose of two or three times or more times per day.

(Subject of administration)

The subject to which the agent or composition of the present invention comprising compound A or a salt thereof is administered is not particularly limited as long as it is an animal that is in need of or wishes for the agent or composition of the present invention, and may be, for example, a mammal such as a human, a pig, a cattle, a horse, a goat, a sheep, a boar, a rabbit, a mouse, a rat, a hamster, a guinea pig, a monkey, a rhesus monkey, a cynomolgus monkey, a marmoset, an orangutan, a chimpanzee, a dog, or a cat, and a human is preferable.

(Combined use)

The agent or composition of the present invention may further comprise one or more active ingredients other than compound A or a salt thereof. Moreover, when administering the composition of the present invention, one or more active ingredients other than compound A or a salt thereof, or an agent comprising it (them), may be administered in combination.

(Use)

The agent or composition of the present invention can be used to, for example, promote energy consumption, increase oxygen consumption, promote lipid burning, promote fat burning, promote thermogenesis, increase body temperature, suppress an increase in body weight, suppress an increase in or reduce body fat percentage, suppress an increase in or reduce body fat mass, suppress body fat accumulation, promote increase muscle mass, enhance muscle, prevent or ameliorate obesity, and/or suppress an increase in or reduce total cholesterol level. Here, the use may be either therapeutic use or non-therapeutic use. "Non-therapeutic" refers to a concept that does not include medical practice, or that is to say, a concept that does not include a method involving surgery, treatment or diagnosis of a human being, and more specifically a concept that does not include a method involving surgery, treatment or diagnosis on a human being performed by a physician or a person under the direction of the physician.

Below, the present invention will now be described more specifically by way of Examples, but the technical scope of the present invention is not limited thereto.

## Examples

1. Test materials and experimental methods

1-1. Test materials

(1) Examined substance

Name: Compound A (compound of the following formula (A))

[Chem. 12]

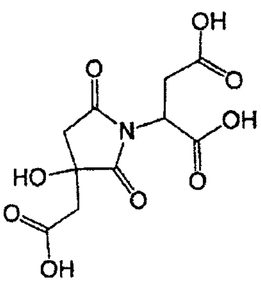

(A)

Production method: Crystalline compound A (a mixture of the steric structure of SS isomer and the steric structure of RR isomer in the RS notation system) was produced in accordance with the method described in WO2018/199040 at paragraphs [0048]-[0049].
Properties: Powdery, white
Storage conditions: Refrigerated
Usage: Predetermined weight was measured, and dispersed in a 0.5% carboxymethyl cellulose (CMC) solution as a solvent to give an oral administration solution.

(2) Control substance

Name: CMC
Manufacturer: Nacalai Tesque, Inc.
Container: 500 mL plastic bottle
Lot number: M0A9874
Properties: Powdery, white
Storage conditions: Refrigerated

Usage: Predetermined weight was measured, and dissolved in Otsuka distilled water to 0.5% to give an oral administration solution.

1-2. Animal used

(1) Species, strain, and sex
Mouse, C57BL/6J, male
(2) Reasons for selecting species and strain The mouse is widely used in a safety test and a pharmacological test of a drug, a medical apparatus, and like, and the mouse of the strain used in this test was selected because its characteristics are well known.
(3) Microorganism control
SPF
(4) Manufacturer
The Jackson Laboratory Japan, Inc.
(5) Number of animals arrived
48
(6) Age at arrival
7 to 8 Weeks old
(7) Body weight at arrival
21 to 28 g
(8) Individual identification method
Numbers were drawn on the tails with an oil-based marker for identification. A number from 1 to 40 was assigned to the respective animals in this study.
(9) Quarantine and acclimatization
No quarantine was performed. After arrival, a period of acclimatization was provided and, during this period, animals were housed in an animal room on ultra high fat diet (D12492) as a solid diet, and their health status was monitored occasionally.

1-3. Housing environment

Temperature: 23 ± 3°C
Humidity: 55 ± 15%
Ventilation frequency: 13 to 16 times/hour
Lighting time: 12 hours (7:00 to 19:00)

1-4. Diet for feeding animals

During the test period, the animals were fed ad libitum ultra high fat diet D12492 (EP Trading Co., Ltd., Lot: 22041308). After the end of the test, the entirety of the test food that the animals were fed was discarded.

Name: D12492 (ultra high fat diet, 60 kcal% fat, blue)
Manufacturer: EP Trading Co., Ltd.
Lot number: 22041308
Usage: Removed from refrigerator when fed.
Composition: Shown in Table 1.

[Table 1]

| Table 1: Composition of diet for feeding animals | | |
|---|---|---|
| Product | D12492 | |
| % | gm% | kcal% |
| Protein | 26 | 20 |

(continued)

| Table 1: Composition of diet for feeding animals | | |
|---|---|---|
| Product | D12492 | |
| % | gm% | kcal% |
| Carbohydrate | 26 | 20 |
| Lipid | 35 | 60 |
| Total | | 100 |
| kcal/gm | 5.24 | |
| | | |
| Material | gm | kcal |
| Casein, 80 mesh | 200 | 800 |
| L-Cystine | 3 | 12 |
| | | |
| Corn starch | 0 | 0 |
| Maltodextrin 10 | 125 | 500 |
| Sucrose | 68.8 | 275 |
| | | |
| Cellulose, BW 200 | 50 | 0 |
| | | |
| Soy bean oil | 25 | 225 |
| Lard | 245 | 2205 |
| | | |
| Mineral Mix S10026 | 10 | 0 |
| Dicalcium phosphate | 13 | 0 |
| Calcium carbonate | 5.5 | 0 |
| Potassium citrate monohydrate | 16.5 | 0 |
| | | |
| Vitamin Mix V10001 | 10 | 40 |
| Choline bitartrate | 2 | 0 |
| | | |
| FD&C Yellow Dye #5 | 0 | 0 |
| FD&C Red Dye #40 | 0 | 0 |
| FD&C Blue Dye #1 | 0.05 | 0 |
| | | |
| Total | 773.85 | 4057 |

1-5. Drinking water

Dedicated tap water was fed ad libitum from a water bottle.

1-6. Group composition

Shown in Table 2. The body weight of animals having no apparent abnormalities on the day of grouping was measured, and the animals were divided into groups according to the body weight by stratified randomization using a

statistical analysis system EXSUS Ver. 10 (EP Croit Co., Ltd.).

[Table 2]

Table 2: Group composition

| Group | Breeding diet | Test substance | Administration route of compound A | Number of animals |
|---|---|---|---|---|
| CON | D12492 | 0.5% CMC | Not applicable | 10 |
| Compound A administered | D12492 | Compound A 720 mg/10mL/kg | Oral administration | 10 |

1-7. Preparation and administration of test substance

(1) Method for preparing test substance

Compound A was weighed, a small amount of 0.5% CMC was added, and the mixture was uniformly pulverized in a porcelain mortar. Then, 0.5% CMC was further added to the pulverized compound A to regulate the concentration to 720 mg/10 mL. After preparation, the test substance was stored under refrigeration.

(2) Method for administering test substance

The test substance was orally administered (10 mL/kg) using an oral probe in a total of twice a day, i.e., once in the morning and once in the afternoon, for 12 weeks. During oral administration, the animal was held by hand.

1-8. Observation

(1) Observation of general conditions

During the administration period of the test substance, the general conditions of the animals were closely observed.

(2) Calculation of calorie intake

Food intake (g) was measured at least once a week from the day of grouping until the time of necropsy, and multiplied by the calorific value (kcal) per weight for each animal to calculate calorie intake.

(3) Analysis of exhaled gas

A 32-channel energy metabolism measurement system for small animals (ARCO-2000-RAT, Arco System Inc.) was used to measure exhaled gas. Oxygen consumption (mL/min) and Carbon dioxide production (mL/min) of the animals were measured every 10 minutes at an air flow rate of 0.3 L/min. Measurements were carried out for about 24 hours after the completion of oral administration in the morning of the day before necropsy, and total oxygen consumption (mL/kg/day), energy consumption (kcal/kg/day), and lipid burning (mg/kg/day) over 24 hours were calculated using the following formulae:
Calculation formulae:

Total oxygen consumption (mL/kg/day) = Mean $VO_2$ over about 24 hours (mL/min) / Body weight (kg) × 60 (min/h) × 24 (h)

Total energy consumption (kcal/kg/day) = [{3.816 × Mean $VO_2$ over about 24 hours (mL/min) + 1.231 × Mean $VCO_2$ over about 24 hours (mL/min) } / Body weight (kg) × 60 (min/h) × 24 (h)] / 1000 (cal/kcal)

Total lipid burning (mg/kg/day) = 1.67 × [Mean $VO_2$ over about 24 hours (mL/min) - Mean $VCO_2$ over about 24 hours (mL/min)] / Body weight (kg) × 60 (min/h) × 24 (h)

($VO_2$: oxygen consumption, $VCO_2$: carbon dioxide production)

(4) Rectal temperature

Before necropsy, a temperature controller for small animals (NS-TC10 Temperature Controller, Neuroscience, Inc.) equipped with a probe for measuring the rectal temperature was inserted into the rectum to measure the rectal temperature.

(5) Body weight measurement

Measurements were carried out at the time of arrival and every week from the day of grouping until the time of necropsy. Measurements were also carried out on the day of measuring the body composition and immediately before necropsy.

(6) Body composition

The body fat mass and the lean body mass were measured with a body composition analyzer (Echo MRI-700, Hitachi, Ltd.) at 6, 9, and 12 weeks of the test food intake, and divided by the body weight to calculate body fat percentage and lean body mass percentage.

1-9. Test method

Animals after arrival were fed ad libitum a solid diet of ultra high fat diet (D12492). After an acclimatization period, the animals were divided into groups according to the body weight. Then, the animals were individually housed and fed ad libitum the ultra high fat diet. The test substance was orally administered to all groups twice daily (once in the morning and once in the afternoon) (CON group: 0.5% CMC was orally administered; compound A administered group: compound A was orally administered). The day of the first administration was regarded as day 0 from the start of the test. Here, 5 animals from each group were regarded as the first-half animals, and the rest were regarded as the second-half animals, and feeding and administration for the second-half animals were started on day 2 from the start of the test of the first-half animals (day 0 from the start of the test of the second-half animals). In order for the animals to be acclimated to the environment inside an exhaled gas measurement chamber, the animals after oral administration in the morning (9:00) at days 7, 21, and 49 days from the start of the test were individually accommodated in the exhaled gas measurement chamber for 5.5 hours (10:00-15:30) with free access to water under fasting conditions. On days 28 and 65, after oral administration in the morning (11:00), all applicable animals were promptly accommodated individually in the exhaled gas measurement chamber, and acclimated for about 24 hours with free access to water and food. Oral administration was carried out in the afternoon of the same day (15:30) and in the morning of the next day (8:30), and the animals after administration were promptly accommodated in the exhaled gas measurement chamber again. After acclimation, the animals were returned to the cages for housing them. On day 82, all applicable animals were promptly accommodated in the exhaled gas measurement chamber (10:00) before oral administration in the morning. Oral administration was carried out in the morning (11:00) and afternoon (15:30) of the same day, and after oral administration in the morning (8:30) of the next day, exhaled gas was measured until necropsy. The animals were subjected to necropsy in the afternoon on day 83 (13:00). At the time of necropsy, laparotomy was performed under isoflurane anesthesia, and blood was collected from the caudal vena cava. After blood collection, the animals were euthanized by bleeding from the site of blood collection, and organs (fat, skeletal muscle) were collected.

1-10. Sample collection

(1) White fat tissue 1: Epididymal fat

At the time of necropsy, fat around the left and right epididymides was taken out, and then the epididymides were discarded. After slight flushing with physiological saline (0.9% aqueous sodium chloride solution, manufactured by Otsuka Pharmaceutical Factory, Inc.) and wiping moisture off, the left and right tissues were weighed together using a precision balance. The right tissue was frozen in liquid nitrogen, and then cryopreserved in a freezer set at -80°C until analysis. The left organ was stored in a 10% neutral buffered formalin solution until analysis.

(2) White fat tissue 2: Mesenteric fat

At the time of necropsy, fat from the stomach to the rectum was taken out. After slight flushing with physiological saline (0.9% aqueous sodium chloride solution, manufactured by Otsuka Pharmaceutical Factory, Inc.) and wiping moisture off, the fat was weighed using a precision balance. The tissue was frozen in liquid nitrogen, and then

cryopreserved in a freezer set at -80°C until analysis.

(3) White fat tissue 3: Perirenal fat

At the time of necropsy, fat around the left and right kidneys was taken out and, after slight flushing with physiological saline (0.9% aqueous sodium chloride solution, manufactured by Otsuka Pharmaceutical Factory, Inc.) and wiping moisture off, the left and right tissues were weighed together using a precision balance. The tissues were frozen in liquid nitrogen, and then cryopreserved in a freezer set at -80°C until analysis.

(4) White fat tissue 4: Interscapular white fat

At the time of necropsy, white fat around the interscapular brown fat was taken out and, after slight flushing with physiological saline (0.9% aqueous sodium chloride solution, Otsuka Pharmaceutical Factory, Inc.) and wiping moisture off, the tissue was weighed using a precision balance. The tissue was frozen in liquid nitrogen, and then cryopreserved in a freezer set at -80°C until analysis.

(5) Skeletal muscle

At the time of necropsy, the plantaris muscle, the anterior tibialis muscle, and the extensor digitorum longus muscle, which are the left and right lower limb skeletal muscles, were collected, and the left and right tissues were weighed together using a precision balance. The tissues were frozen in liquid nitrogen, and then cryopreserved in a freezer set at -80°C until analysis.

(6) Blood

At the time of necropsy, laparotomy was performed under isoflurane anesthesia, and about 0.5 mL of blood was collected from the caudal vena cava, introduced into heparin-containing plasma separation blood collection tubes (BD Microtainer PSTTM Tubes with Lithium Heparin 365985 (yellow green); Nippon Becton Dickinson Co., Ltd.), and temporarily stored on ice. Blood was centrifuged (3000 rpm, 10 min, 4°C), and the supernatant plasma was transferred to a 1.5 mL microtube (1.5 mL Hyper Microtube Round-Bottom Hard Touch 131-715C, WATSON CO., LTD), and cryopreserved in a freezer set at -80°C until analysis. Measurement of total cholesterol in plasma was assigned to the Nagahama Life Science Laboratory of Oriental Yeast Co., Ltd., Nagahama Office.

1-11. Data aggregation and statistical analysis

Data is expressed as mean ± Standard Deviation. Concerning statistical processing, a two-sample equal variance t-test (two-sided test) was performed to make comparisons between groups using the CON group as a control. In all cases, the significance level was set at 5%. Excel (2013 ver. Microsoft) was used in an analysis thereof.

2. Results

2-1. Calorie intake

There was no difference between the groups in the mean daily calorie intake during the test period (Table 3, Figure 1). Concerning one animal in the CON group, data was missing due to measurement error and, accordingly, quantification was performed without the corresponding data (food intake on days 4 and 5).

[Table 3]

Table 3: Calorie intake

| | CON (n=10) | | | Compound A administered (n=10) | | |
|---|---|---|---|---|---|---|
| | mean | ± | SD | mean | ± | SD |
| Calorie intake (kcal/day) | 11.1 | ± | 1.1 | 10.7 | ± | 0.3 |

2-2. Oxygen consumption / Energy consumption / lipid burning

The compound A administered group had total oxygen consumption, total energy consumption, and total lipid

burning over 24 hours from the start of administration to the end of measurement (11:32 to 11:32) significantly higher than those of the CON group (Table 4, Figures 2 to 5). This result showed that compound A has an effect of increasing oxygen consumption, energy consumption, and lipid burning.

[Table 4]

Table 4: total oxygen consumption, total carbon dioxide production, total energy consumption, and total lipid burning over 24 hours

| | CON (n=10) | | | Compound A administered (n=10) | | |
|---|---|---|---|---|---|---|
| | mean | ± | SD | mean | ± | SD |
| total oxygen consumption (mL/kg/day) | 45697 | ± | 3929 | 51382 | ± | 2777** |
| total carbon dioxide production (mL/kg/day) | 37509 | ± | 4995 | 40719 | ± | 2597 |
| total energy consumption (kcal/kg/day) | 219 | ± | 19 | 243 | ± | 17** |
| total lipid burning (mg/kg/day) | 14986 | ± | 1692 | 17768 | ± | 1526** |
| ** p < 0.01; Compound A administered vs CON. (t-test) | | | | | | |

2-3. Rectal temperature

The compound A administered group was recognized as having a significantly higher rectal temperature than that of the CON group (Table 5, Figure 6). This result showed that intake of compound A promoted thermogenesis in the mouse body and increased the body temperature. That is to say, compound A was shown to have a thermogenesis promoting effect and a body temperature increasing effect.

[Table 5]

Table 5: Rectal temperature

| | CON (n=10) | | | Compound A administered (n=10) | | |
|---|---|---|---|---|---|---|
| | mean | ± | SD | mean | ± | SD |
| Rectal temperature (°C) | 33.7 | ± | 1.1 | 35.7 | ± | 1.0** |
| ** p < 0.01; Compound A administered vs CON.(t-test) | | | | | | |

2-4. Body weight

Concerning the body weight change during the test food administration period, the compound A administered group tended to have a lower body weight than that of the CON group at 7, 8, and 9 weeks from the start of the test, and recognized as having a significantly lower value at 10, 11 and 12 weeks (Table 6, Figure 7). This result showed that compound A has a body weight increase suppressing effect.

For example, after 12 weeks of administration, the mean body weight gain in the CON group was 7.2 g, while the mean body weight gain in the compound A administered group was 3.8 g. That is to say, relative to the body weight gain of the CON group being 100%, the body weight gain of the compound A administered group was 52.8 ((3.8 / 7.2) × 100)%.

[Table 6]

Table 6: Body weight

| | CON (n=10) | | | Compound A administered (n=10) | | | |
|---|---|---|---|---|---|---|---|
| | mean | ± | SD | mean | ± | SD | |
| Before administration | 25.4 | ± | 1.2 | 25.1 | ± | 0.8 | |
| 1 week (g) | 25.1 | ± | 1.4 | 24.9 | ± | 1.0 | |
| 2 weeks (g) | 25.7 | ± | 1.7 | 25.1 | ± | 1.1 | |
| 3 weeks (g) | 26.0 | ± | 2.0 | 25.3 | ± | 1.1 | |
| 4 weeks (g) | 26.7 | ± | 2.5 | 25.4 | ± | 1.1 | |
| 5 weeks (g) | 27.5 | ± | 3.1 | 25.8 | ± | 1.2 | |
| 6 weeks (g) | 28.4 | ± | 3.8 | 26.4 | ± | 1.2 | |
| 7 weeks (g) | 29.2 | ± | 3.9 | 26.8 | ± | 1.4 | p=0.087 |

(continued)

Table 6: Body weight

| | CON (n=10) | | | Compound A administered (n=10) | | | |
|---|---|---|---|---|---|---|---|
| | mean | ± | SD | mean | ± | SD | |
| 8 weeks (g) | 30.1 | ± | 4.5 | 27.3 | ± | 1.7 | p=0.082 |
| 9 weeks (g) | 31.1 | ± | 5.1 | 27.8 | ± | 1.8 | p=0.068 |
| 10 weeks (g) | 31.2 | ± | 4.9 | 27.2 | ± | 2.1* | |
| 11 weeks (g) | 32.4 | ± | 5.3 | 28.3 | ± | 1.8* | |
| 12 weeks (g) | 32.6 | ± | 4.8 | 28.9 | ± | 1.3* | |

* p < 0.05; Compound A administered vs CON (t-test).

2-5. Body fat percentage and lean body mass percentage

Concerning body fat percentage and lean body mass percentage, the compound A administered group at 6 weeks of the test food intake tended to have a lower body fat percentage and a higher lean body mass percentage than those of the CON group, and the compound A administered group at 9 and 12 weeks had a significantly lower body fat percentage and higher lean body mass percentage (Table 7, Figure 8). This result showed that compound A has a body fat percentage increase suppressing effect (i.e., a lean body mass percentage decrease inhibiting effect).

[Table 7]

Table 7: Body fat percentage and Lean body mass percentage

| | CON (n=10) | | | Compound A administered (n=10) | | | |
|---|---|---|---|---|---|---|---|
| | mean | ± | SD | mean | ± | SD | |
| Body fat percentage (%) | | | | | | | |
| 6 weeks | 19.4 | ± | 6.0 | 15.6 | ± | 3.3 | p=0.092 |
| 9 weeks | 23.8 | ± | 8.0 | 17.7 | ± | 3.9* | |
| 12 weeks | 26.9 | ± | 7.4 | 19.0 | ± | 3.8** | |
| Lean body mass percentage (%) | | | | | | | |
| 6 weeks | 75.6 | ± | 5.6 | 79.6 | ± | 2.9 | p=0.057 |
| 9 weeks | 71.3 | ± | 7.8 | 77.3 | ± | 3.9* | |
| 12 weeks | 67.9 | ± | 6.6 | 74.7 | ± | 3.4** | |

* p <0.05, ** p < 0.01; Compound A administered vs CON (t-test).

2-6. White fat weight

Concerning the white fat weight per body weight, the compound A administered group had significantly lower values of epididymal fat, mesenteric fat, perirenal fat, and interscapular fat than that of the CON group (Table 8, Figure 9). This result showed that compound A has an effect of suppressing an increase in white fat weight per body weight.

[Table 8]

Table 8: White fat weight

| | CON (n=10) | | | Compound A administered (n=10) | | |
|---|---|---|---|---|---|---|
| | mean | ± | SD | mean | ± | SD |
| Epididymal fat (g/Kg body weight) | 49.92 | ± | 13.91 | 32.18 | ± | 6.20** |
| Mesenteric fat (g/Kg body weight) | 13.14 | ± | 3.22 | 10.10 | ± | 1.43* |
| Perirenal fat (g/Kg body weight) | 20.02 | ± | 5.49 | 12.12 | ± | 2.08** |
| Interscapular fat (g/Kg body weight) | 2.57 | ± | 0.74 | 1.88 | ± | 0.40* |

* p<0.05, ** p < 0.01; Compound A administered vs CON (t-test).

2-7. Muscle weight

Concerning the weight per body weight, the compound A administered group was recognized as having significantly greater weights of the plantaris muscle, the anterior tibialis muscle, and the extensor digitorum longus muscle, as well as a significantly greater total weight of these three types of muscle (Table 9, Figure 10). This result showed that compound A has an effect of promoting an increase in muscle weight per body weight.

[Table 9]

Table 9: Muscle weight

| | CON (n=10) | | | Compound A administered (n=10) | | |
|---|---|---|---|---|---|---|
| | mean | ± | SD | mean | ± | SD |
| Plantaris muscle (g/Kg body weight) | 1.19 | ± | 0.18 | 1.31 | ± | 0.06* |
| Anterior tibialis muscle (g/Kg body weight) | 3.17 | ± | 0.38 | 3.53 | ± | 0.23* |
| Extensor digitorum longus muscle (g/Kg body weight) | 0.76 | ± | 0.09 | 0.84 | ± | 0.06* |
| Muscles (g/Kg body weight) | 5.12 | ± | 0.63 | 5.68 | + | 0.24* |

* $p<0.05$; Compound A administered vs CON (t-test).
Muscles: Total weight of 3 types of muscle, i.e., plantaris muscle, anterior tibial muscle, and extensor digitorum longus muscle.

2-8. Total cholesterol level

The compound A administered group was recognized as having a significantly lower value of total cholesterol in plasma than that of the CON group (Table 10, Figure 11). This result showed that compound A has a total cholesterol level increase suppressing effect.

[Table 10]

Table 10: Total cholesterol level

| | CON (n=10) | | | Compound A administered (n=10) | | |
|---|---|---|---|---|---|---|
| | mean | + | SD | mean | + | SD |
| Total cholesterol (mg/dL) | 174.2 | + | 13.3 | 149.4 | + | 13.2** |

** $p < 0.01$; Compound A administered vs CON (t-test).

**Industrial Applicability**

The present invention can be used particularly effectively in the field of food and pharmaceutical production.

**Claims**

1. An agent for promoting energy consumption, comprising a compound of the following formula (A) or a salt thereof as an active ingredient.

[Chem. 1]

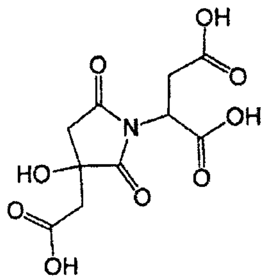

(A)

2. The agent for promoting energy consumption according to claim 1, wherein the agent promotes energy consumption by increasing oxygen consumption.

3. An agent for promoting lipid burning, comprising a compound of the following formula (A) or a salt thereof as an active ingredient.

[Chem. 2]

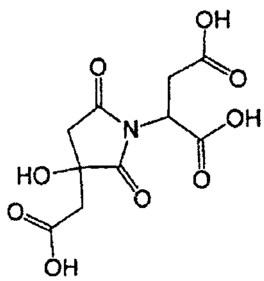

(A)

4. The agent for promoting lipid burning according to claim 3, wherein the agent is for promoting fat burning.

5. An agent for promoting thermogenesis, comprising a compound of the following formula (A) or a salt thereof as an active ingredient.

[Chem. 3]

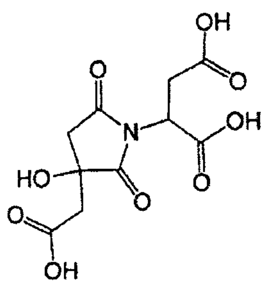

(A)

6. The agent for promoting thermogenesis according to claim 5, wherein the agent is for increasing body temperature.

**7.** An agent for suppressing an increase in body weight, comprising a compound of the following formula (A) or a salt thereof as an active ingredient.

[Chem. 4]

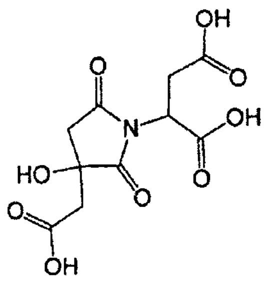

(A)

**8.** An agent for suppressing an increase in or for reducing body fat percentage, comprising a compound of the following formula (A) or a salt thereof as an active ingredient.

[Chem. 5]

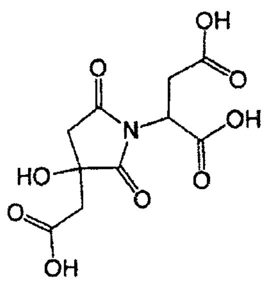

(A)

**9.** The agent for suppressing an increase in or for reducing body fat percentage according to claim 8, wherein the agent is for suppressing an increase in body fat mass.

**10.** The agent for suppressing an increase in or for reducing body fat percentage according to claim 8, wherein the agent is for suppressing body fat accumulation.

**11.** An agent for enhancing muscle, comprising a compound of the following formula (A) or a salt thereof as an active ingredient.

[Chem. 6]

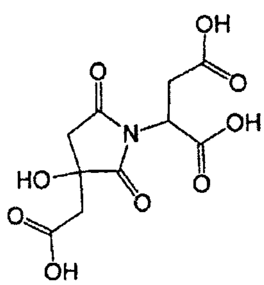

(A)

**12.** The agent for enhancing muscle according to claim 11, wherein the agent is for promoting an increase in muscle mass.

13. An agent for preventing or ameliorating obesity, comprising a compound of the following formula (A) or a salt thereof as an active ingredient.

[Chem. 7]

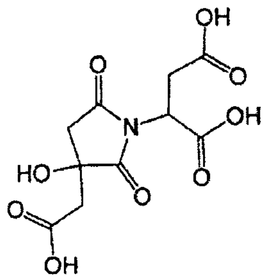

(A)

14. An agent for suppressing an increase in or for reducing total cholesterol level, comprising a compound of the following formula (A) or a salt thereof as an active ingredient.

[Chem. 8]

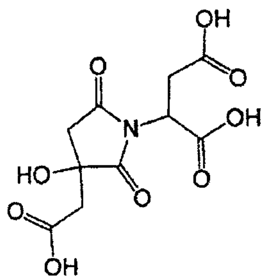

(A)

15. A pharmaceutical composition or a food composition, comprising the agent according to any one of claims 1 to 14.

[Fig. 1]

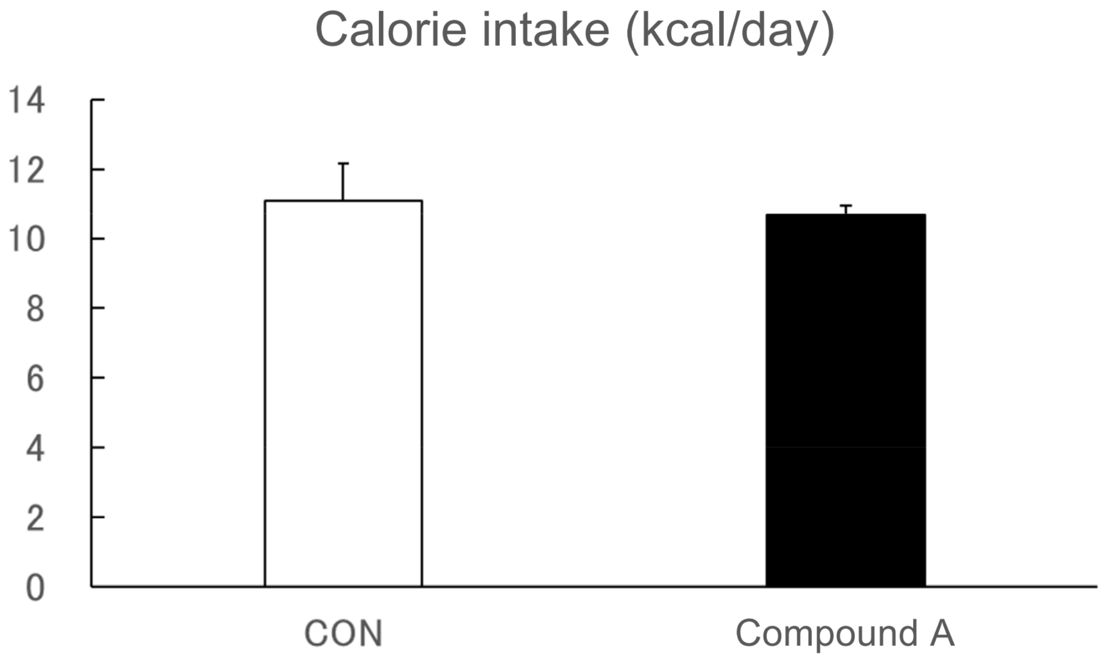


Data are mean ± SD , CON: n = 10, Compound A: n=10.

[Fig. 2]

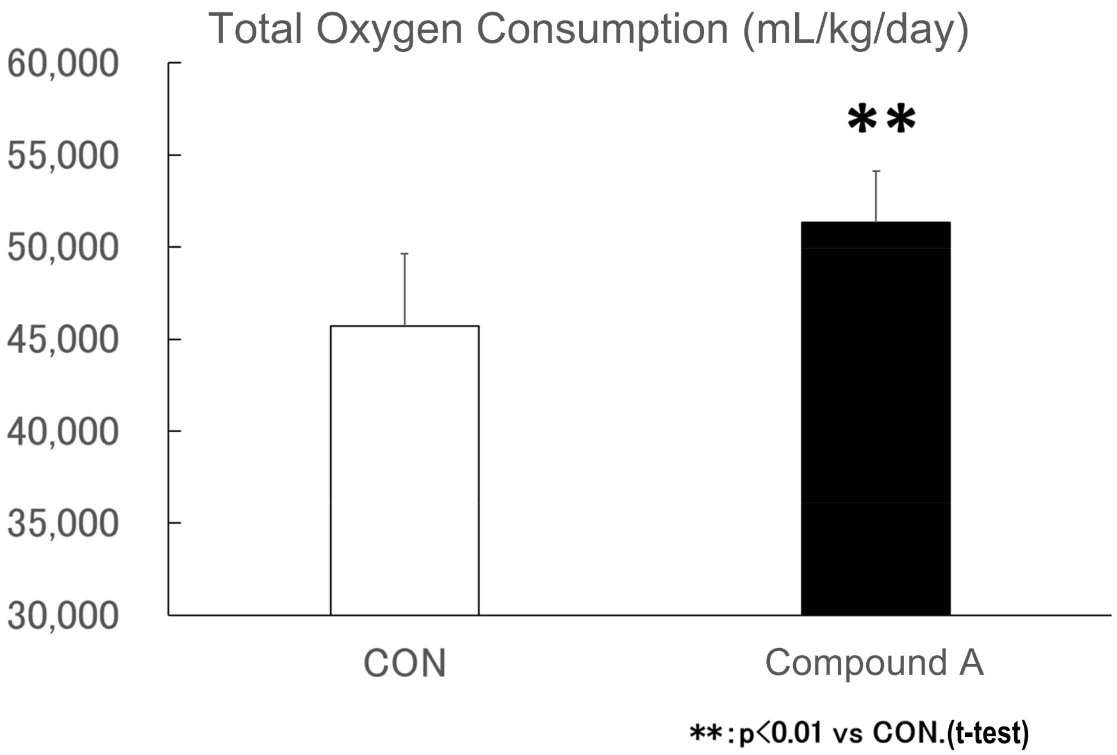


Data are mean ± SD , CON: n=10, Compound A: n=10.
** $p < 0.01$; Compound A vs CON.(t-test)

[Fig. 3]

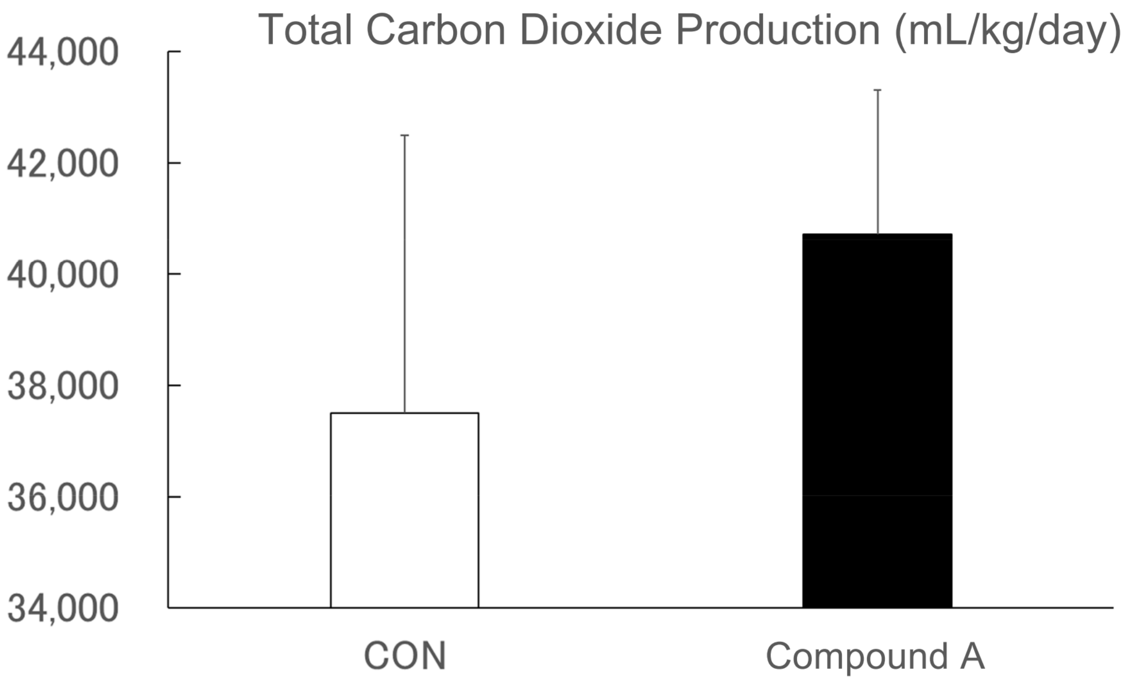


Data are mean ± SD , CON: n=10, Compound A : n=10.

[Fig. 4]

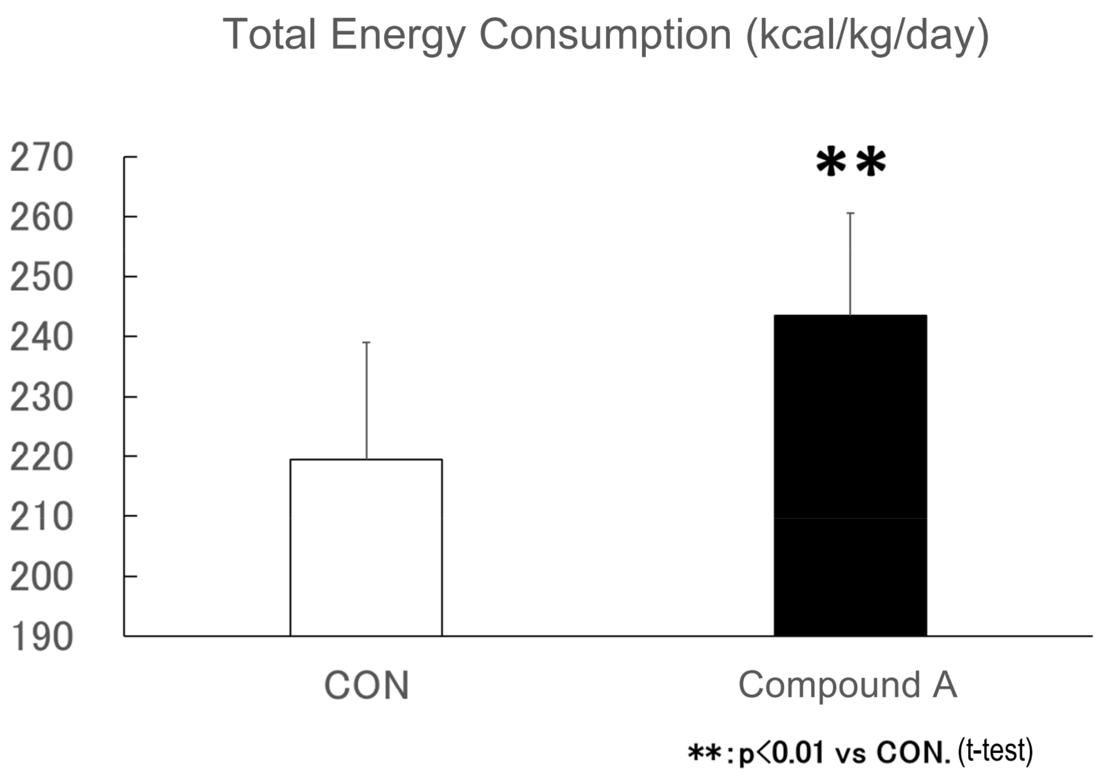


Data are mean ± SD , CON: n = 10, Compound A: n=10.
** $p < 0.01$; Compound A vs CON.(t-test)

[Fig. 5]

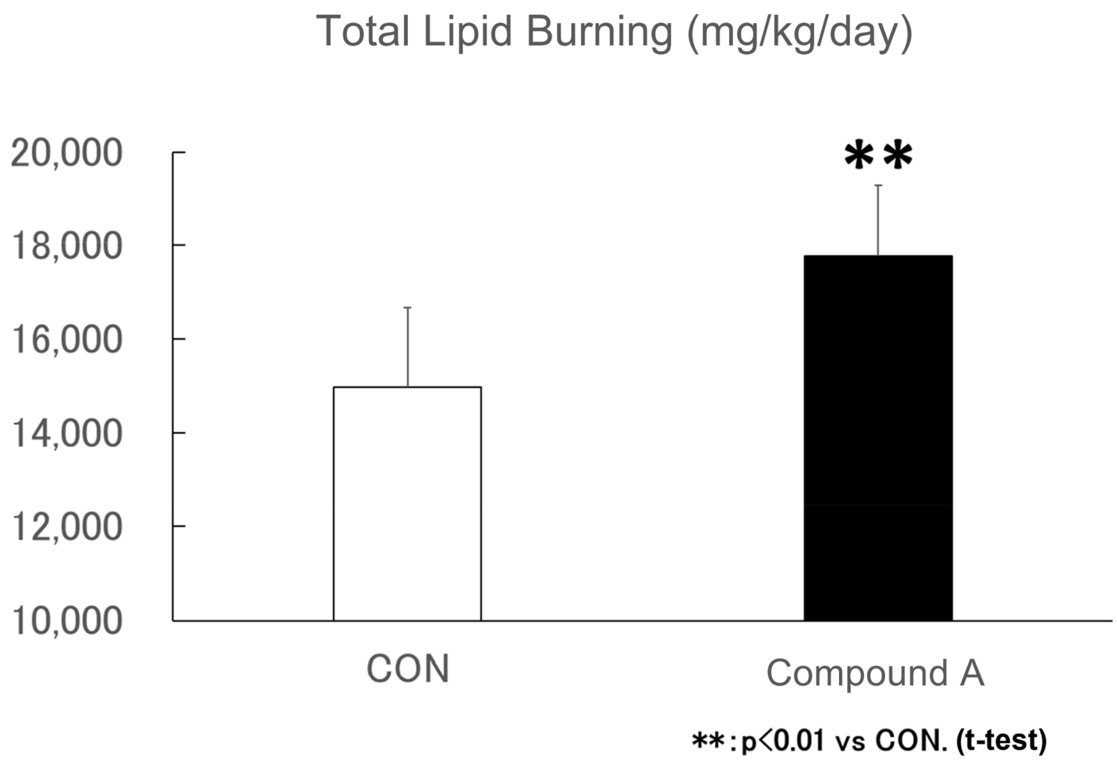


Data are mean ± SD , CON: n = 10, Compound A: n=10.
** $p < 0.01$; Compound A vs CON. (t-test)

[Fig. 6]

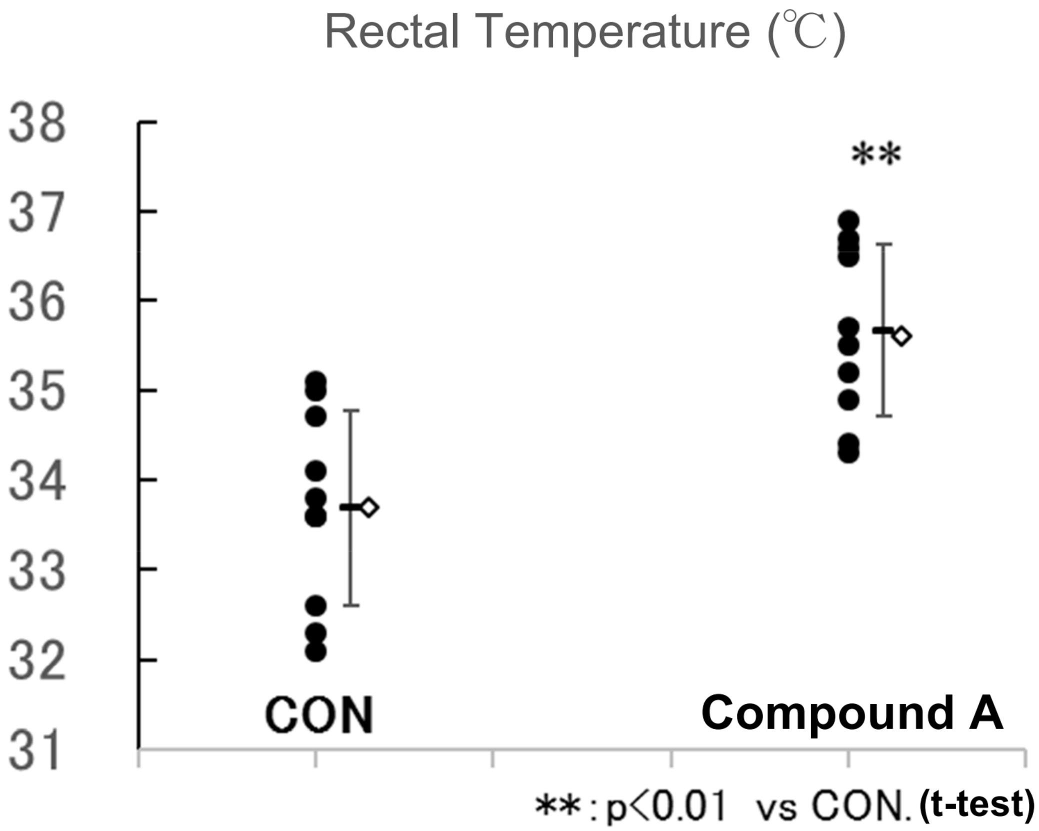


Data are mean ± SD , CON: n = 10, Compound A: n=10.
** p < 0.01; Compoun A vs CON. (t-test)

[Fig. 7]

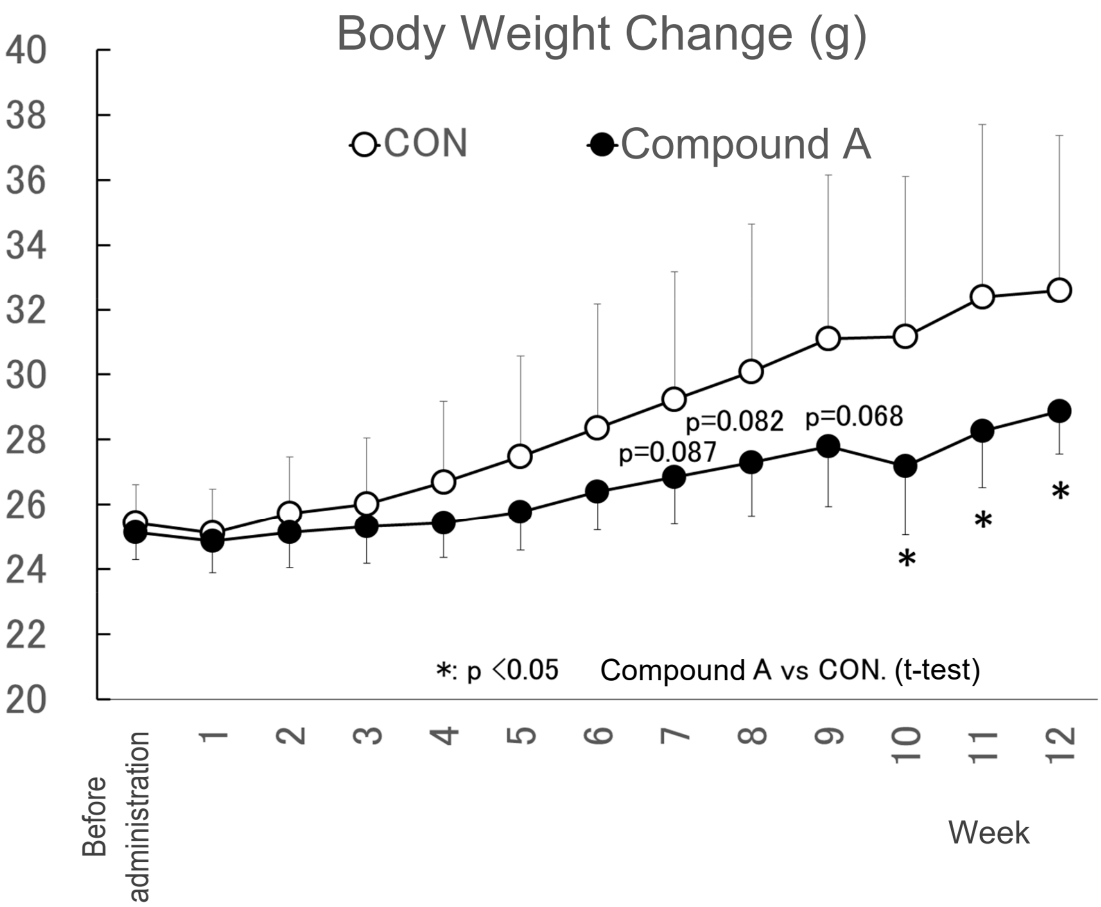


Data are mean ± SD , CON: n=10, Compound A: n=10.
* $p < 0.05$; Compound A vs CON (t-test).

[Fig. 8]

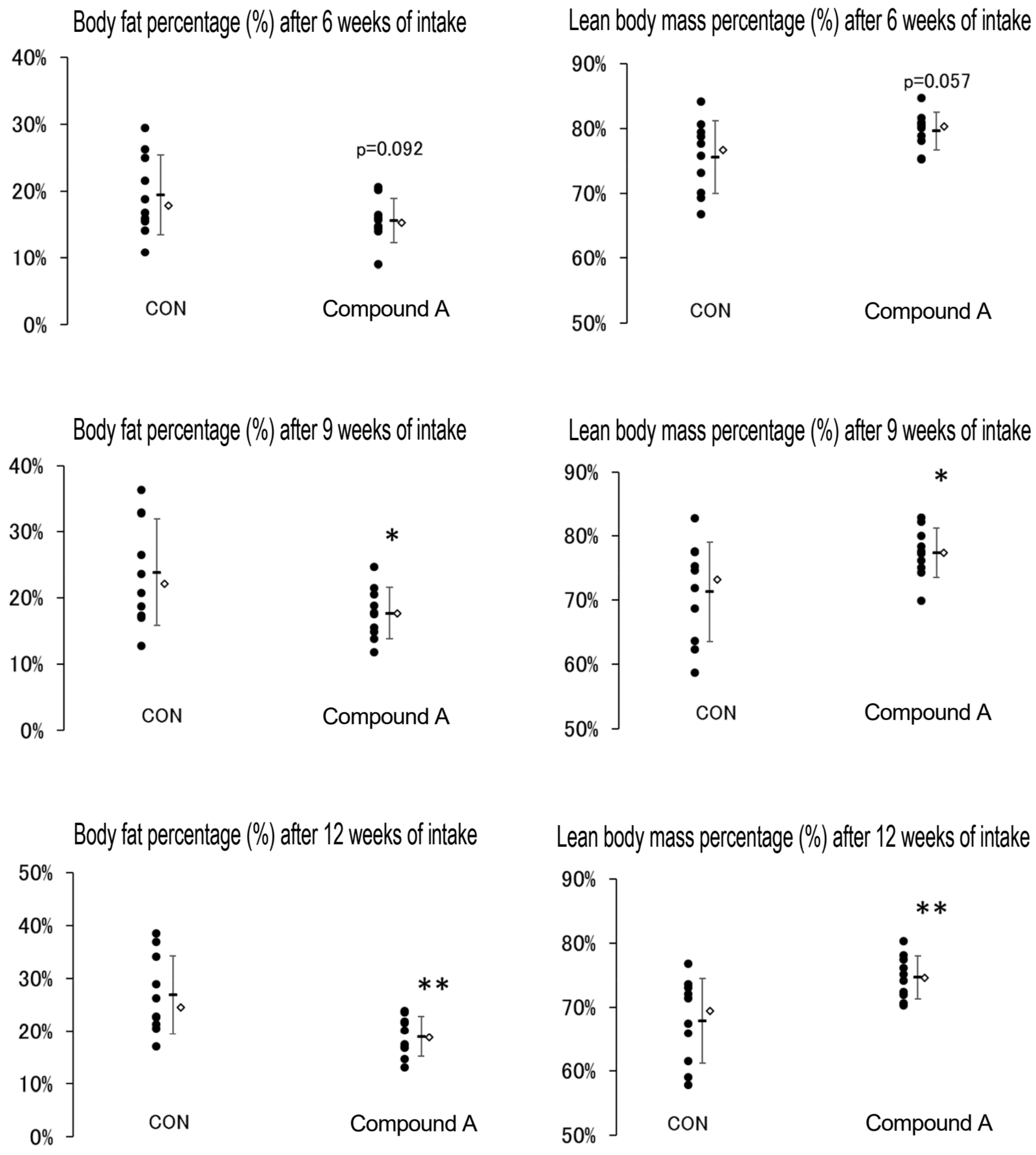


Data are mean ± SD, ◇ are Median . CON: n=10, Compound A: n=10.
* p <0.05, ** p<0.01; Compound A vs CON (t-test)

[Fig. 9]

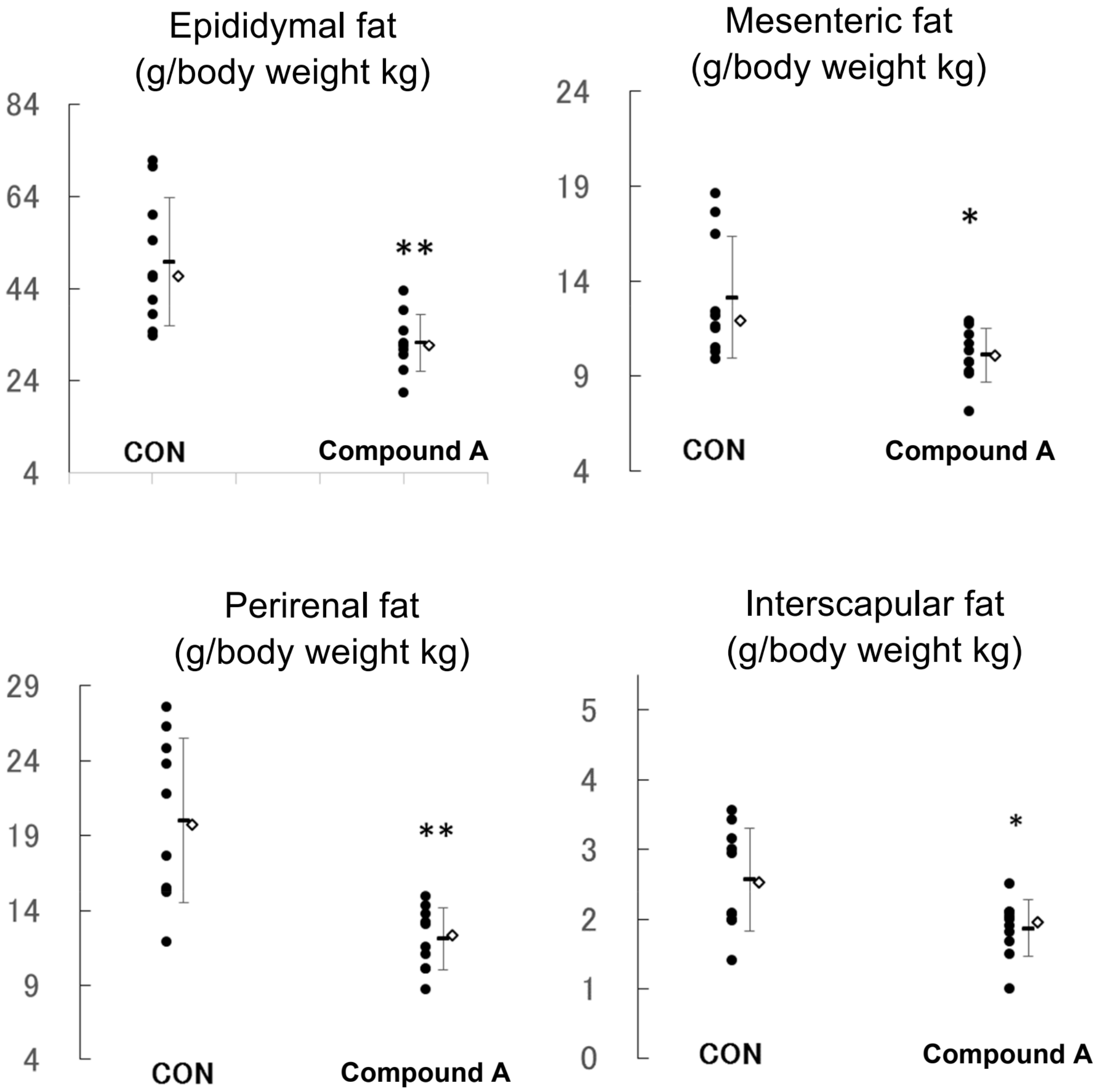


Data are mean ± SD, ◇ are Median . CON: n = 10, Compound A: n=10.
* p < 0.05, ** p < 0.01; Compound A vs CON. (t-test)

[Fig. 10]

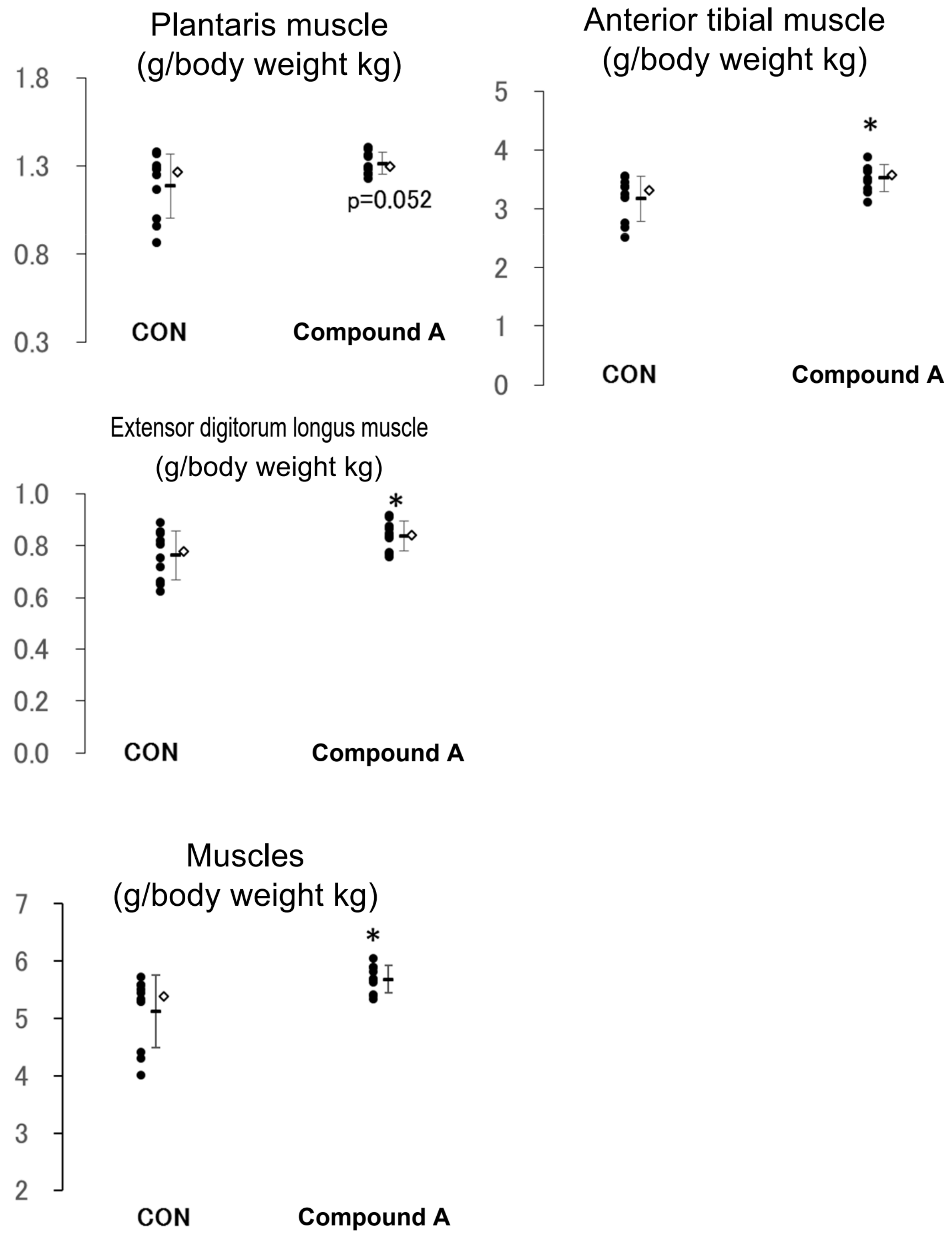


Data are mean ± SD, ◇ are Median . CON: n = 10, Compound A: n=10.
* $p < 0.05$; Compound A vs CON. (t-test)
Muscles: Total weight of 3 types of muscle, i.e., plantaris muscle, anterior tibial muscle, and extensor digitorum longus muscle.

[Fig. 11]

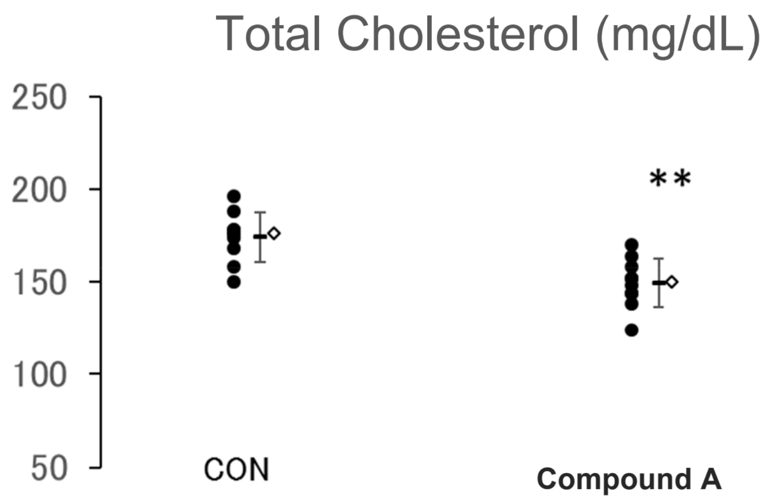


Data are mean ± SD, ◇ are Median . CON: n = 10, Compound A: n=10.
** $p < 0.01$; Compound A vs CON (t-test).

## INTERNATIONAL SEARCH REPORT

International application No.
**PCT/JP2024/021602**

### A. CLASSIFICATION OF SUBJECT MATTER

***A61K 31/4015***(2006.01)i; ***A23L 33/10***(2016.01)i; ***A61P 3/00***(2006.01)i; ***A61P 3/04***(2006.01)i; ***A61P 3/06***(2006.01)i; ***A61P 21/00***(2006.01)i
FI: A61K31/4015; A23L33/10; A61P3/00; A61P3/04; A61P3/06; A61P21/00

According to International Patent Classification (IPC) or to both national classification and IPC

### B. FIELDS SEARCHED

Minimum documentation searched (classification system followed by classification symbols)

A61K31/4015; A23L33/10; A61P3/00; A61P3/04; A61P3/06; A61P21/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| A | WO 2017/077707 A1 (ADABIO CO., LTD.) 11 May 2017 (2017-05-11) | 1, 2, 15 |
| A | WO 2018/199040 A1 (OTSUKA PHARMACEUTICAL FACTORY, INC.) 01 November 2018 (2018-11-01) | 1, 2, 15 |
| A | WO 2020/096002 A1 (KAGAWA UNIVERSITY) 14 May 2020 (2020-05-14) | 1, 2, 15 |
| A | JP 2022-165316 A (KYOTO UNIVERSITY) 31 October 2022 (2022-10-31) | 1, 2, 15 |
| A | JP 2010-241800 A (KAO CORPORATION) 28 October 2010 (2010-10-28) | 1, 2, 15 |

☐ Further documents are listed in the continuation of Box C.    ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **23 August 2024** | **03 September 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No. **PCT/JP2024/021602**

**Box No. III Observations where unity of invention is lacking (Continuation of item 3 of first sheet)**

This International Searching Authority found multiple inventions in this international application, as follows:

(Invention 1) Claims 1, 2, and 15 (parts referring to any of claims 1 and 2)

Claims 1, 2, and 15 have the special technical feature of an energy consumption accelerator containing a compound represented by formula (A) or a salt thereof as an active ingredient, and are thus classified as invention 1.

(Invention 2) Claims 3, 4, and 15 (parts referring to any of claims 3 and 4)

Claims 3, 4, and 15 share, with claim 1 classified as invention 1, the common technical feature of an invention of a formulation containing a compound represented by formula (A) or a salt thereof as an active ingredient.

However, the technical feature does not make a contribution over the prior art in light of the disclosure of document 1 ([Test Example 1]), and thus cannot be said to be a special technical feature. Also, there are no other the same as or corresponding special technical features between these inventions.

In addition, claims 3, 4, and 15 are not dependent on claim 1. Furthermore, claims 3, 4, and 15 are not substantially identical to or similarly closely related to any of the claims classified as invention 1.

Thus, claims 3, 4, and 15 cannot be classified as invention 1.

(Invention 3) Claims 5, 6, and 15 (parts referring to any of claims 5 and 6)

Claims 5, 6, and 15 share, with claim 1 classified as invention 1, the common technical feature of an invention of a formulation containing a compound represented by formula (A) or a salt thereof as an active ingredient.

However, the technical feature does not make a contribution over the prior art in light of the disclosure of document 1 ([Test Example 1]), and thus cannot be said to be a special technical feature. Also, there are no other the same as or corresponding special technical features between these inventions.

In addition, claims 5, 6, and 15 are not dependent on claim 1. Furthermore, claims 5, 6, and 15 are not substantially identical to or similarly closely related to any of the claims classified as invention 1.

Thus, claims 5, 6, and 15 cannot be classified as invention 1.

(Invention 4) Claims 7 and 15 (parts referring to claim 7)

Claims 7 and 15 share, with claim 1 classified as invention 1, the common technical feature of an invention of a formulation containing a compound represented by formula (A) or a salt thereof as an active ingredient.

However, the technical feature does not make a contribution over the prior art in light of the disclosure of document 1 ([Test Example 1]), and thus cannot be said to be a special technical feature. Also, there are no other the same as or corresponding special technical features between these inventions.

In addition, claims 7 and 15 are not dependent on claim 1. Furthermore, claims 7 and 15 are not substantially identical to or similarly closely related to any of the claims classified as invention 1.

Therefore, claims 7 and 15 cannot be classified as invention 1.

(Invention 5) Claims 8-10 and 15 (parts referring to any of claims 8-10)

Claims 8-10 and 15 share, with claim 1 classified as invention 1, the common technical feature of an invention of a formulation containing a compound represented by formula (A) or a salt thereof as an active ingredient.

However, the technical feature does not make a contribution over the prior art in light of the disclosure of document 1 ([Test Example 1]), and thus cannot be said to be a special technical feature. Also, there are no other the same as or corresponding special technical features between these inventions.

In addition, claims 8-10 and 15 are not dependent on claim 1. Furthermore, claims 8-10 and 15 are not substantially identical to or similarly closely related to any of the claims classified as invention 1.

Thus, claims 8-10 and 15 cannot be classified as invention 1.

(Invention 6) claims 11, 12, and 15 (parts referring to any of claims 11 and 12)

Claims 11, 12, and 15 share, with claim 1 classified as invention 1, the common technical feature of an invention of a formulation containing a compound represented by formula (A) or a salt thereof as an active ingredient.

However, the technical feature does not make a contribution over the prior art in light of the disclosure of document 1 ([Test Example 1]), and thus cannot be said to be a special technical feature. Also, there are no other the same as or corresponding special technical features between these inventions.

In addition, claims 11, 12, and 15 are not dependent on claim 1. Furthermore, claims 11, 12, and 15 are not substantially identical to or similarly closely related to any of the claims classified as invention 1.

Thus, claims 11, 12, and 15 cannot be classified as invention 1.

(Invention 7) Claims 13 and 15 (parts referring to claim 13)

**INTERNATIONAL SEARCH REPORT**

International application No.
**PCT/JP2024/021602**

**Box No. III Observations where unity of invention is lacking (Continuation of item 3 of first sheet)**

Claims 13 and 15 share, with claim 1 classified as invention 1, the common technical feature of an invention of a formulation containing a compound represented by formula (A) or a salt thereof as an active ingredient. However, the technical feature does not make a contribution over the prior art in light of the disclosure of document 1 ([Test Example 1]), and thus cannot be said to be a special technical feature. Also, there are no other the same as or corresponding special technical features between these inventions.

In addition, claims 13 and 15 are not dependent on claim 1. Furthermore, claims 13 and 15 are not substantially identical to or similarly closely related to any of the claims classified as invention 1.

Therefore, claims 13 and 15 cannot be classified as invention 1.

(Invention 8) Claims 14 and 15 (parts referring to claim 14)

Claims 14 and 15 share, with claim 1 classified as invention 1, the common technical feature of an invention of a formulation containing a compound represented by formula (A) or a salt thereof as an active ingredient. However, the technical feature does not make a contribution over the prior art in light of the disclosure of document 1 ([Test Example 1]), and thus cannot be said to be a special technical feature. Also, there are no other the same as or corresponding special technical features between these inventions.

In addition, claims 14 and 15 are not dependent on claim 1. Furthermore, claims 14 and 15 are not substantially identical to or similarly closely related to any of the claims classified as invention 1.

Therefore, claims 14 and 15 cannot be classified as invention 1.

Document 1: WO 2017/077707 A1 (ADABIO CO., LTD.) 11 May 2017 (2017-05-11) & US 2018/0334452 A1, test example, & EP 3372578 A1, & KR 10-2018-0059550 A, & CN 108349877 A

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **Claims 1, 2, and 15 (parts referring to claims 1 and 2)**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

**INTERNATIONAL SEARCH REPORT**
**Information on patent family members**

International application No.
**PCT/JP2024/021602**

| Patent document cited in search report | Publication date (day/month/year) | Patent family member(s) | Publication date (day/month/year) |
|---|---|---|---|
| WO 2017/077707 A1 | 11 May 2017 | US 2018/0334452 A1 | |
| | | EP 3372578 A1 | |
| | | KR 10-2018-0059550 A | |
| | | CN 108349877 A | |
| WO 2018/199040 A1 | 01 November 2018 | US 2020/0055821 A1 | |
| | | EP 3617191 A1 | |
| | | KR 10-2019-0129965 A | |
| | | CN 110546137 A | |
| WO 2020/096002 A1 | 14 May 2020 | US 2021/0386767 A1 | |
| | | EP 3878457 A1 | |
| | | CN 113015533 A | |
| | | KR 10-2021-0091211 A | |
| JP 2022-165316 A | 31 October 2022 | (Family: none) | |
| JP 2010-241800 A | 28 October 2010 | US 2012/0010285 A1 | |
| | | WO 2010/106798 A1 | |
| | | EP 2409696 A1 | |
| | | CN 102355898 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2012020941 A
- JP 2018058832 A
- WO 2017077707 A
- WO 2018199040 A
- WO 201707707 A
- WO 201819940 A